# EUROPEAN PATENT APPLICATION

(11) **EP 2 078 729 A1**
(43) Date of publication of application: **15.07.2009**
(21) Application number: 09155624.1
(22) Date of filing: 30.01.2006
(51) Int. Cl.: C07K 14/575, A61K 38/22, A61K 47/48

(54) **PPY agonists and uses thereof**

(30) Priority: 04.02.2005 US 650366 P; 04.11.2005 US 733656 P
(62) Divisional of application: 06710361.4
(71) Applicant: Pfizer Products Inc., Groton, CT 06340-5146 (US)
(72) Inventor: Finn, Rory Francis, Chesterfield, MO 63017-1732 (US); Siegel, Ned Roger, Chesterfield, MO 63017-1732 (US); Summers, Neena Lynne, St Charles, MO 63304-2423 (US); Nardone, Nancy Ann, Groton, CT 06340 (US)
(74) Representative: England, Peter Michael

(57) **Abstract**

The invention provides PYY₃₋₃₆ variants and pegylated derivatives thereof and compositions and methods useful in the treatment of conditions modulated by an NPY Y2 receptor agonist.

## Description

### Field of the Invention

The present invention relates to PYY agonists, more particularly to PYY₃₋₃₆ variants and to pegylated derivatives of PYY₃₋₃₆ and PYY₃₋₃₆ variants, to compositions containing such agonists, isolated nucleic acids encoding such PYY agonists, and to the use of such agonists or compositions in the treatment of obesity and co-morbidities thereof, or to decrease appetite, food intake or caloric intake in a mammal.

### Background of the Invention

Obesity is a major public health concern because of its increasing prevalence and associated health risks. Moreover, obesity may affect a person's quality of life through limited mobility and decreased physical endurance as well as through social, academic and job discrimination.

Obesity and being overweight are generally defined by body mass index (BMI), which is correlated with total body fat and serves as a measure of the risk of certain diseases. BMI is calculated by weight in kilograms divided by height in meters squared (kg/m²). Overweight is typically defined as a BMI of 25-29.9 kg/m², and obesity is typically defined as a BMI of 30 kg/m² or higher. See, e.g., National Heart, Lung, and Blood Institute, Clinical Guidelines on the Identification, Evaluation, and Treatment of Overweight and Obesity in Adults, The Evidence Report, Washington, DC: U.S. Department of Health and Human Services, NIH publication no. 98-4083 (1998).

Recent studies have found that obesity and its associated health risks are not limited to adults, but also affect children and adolescents to a startling degree. According to the Center for Disease Control, the percentage of children and adolescents who are defined as overweight has more than doubled since the early 1970s, and about 15 percent of children and adolescents are now overweight. Risk factors for heart disease, such as high cholesterol and high blood pressure, occur with increased frequency in overweight children and adolescents compared with normal-weight subjects of similar age. Also, type 2 diabetes, previously considered an adult disease, has increased dramatically in children and adolescents. Overweight conditions and obesity are closely linked to type 2 diabetes. It has recently been estimated that overweight adolescents have a 70% chance of becoming overweight or obese adults. The probability increases to about 80% if at least one parent is overweight or obese. The most immediate consequence of being overweight as perceived by children themselves is social discrimination.

Overweight or obese individuals are at increased risk for ailments such as hypertension, dyslipidemia, type 2 (non-insulin dependent) diabetes, insulin resistance, glucose intolerance, hyperinsulinemia, coronary heart disease, angina pectoris, congestive heart failure, stroke, gallstones, cholescystitis, cholelithiasis, gout, osteoarthritis, obstructive sleep apnea and respiratory problems, gall bladder disease, certain forms of cancer (e.g., endometrial, breast, prostate, and colon) and psychological disorders (such as depression, eating disorders, distorted body image and low self esteem). The negative health consequences of obesity make it the second leading cause of preventable death in the United States and impart a significant economic and psychosocial effect on society. See, McGinnis M, Foege WH., "Actual Causes of Death in the United States," JAMA 270:2207-12, 1993.

Obesity is now recognized as a chronic disease that requires treatment to reduce its associated health risks. Although weight loss is an important treatment outcome, one of the main goals of obesity management is to improve cardiovascular and metabolic values to reduce obesity-related morbidity and mortality. It has been shown that 5-10% loss of body weight can substantially improve metabolic values, such as blood glucose, blood pressure, and lipid concentrations. Hence, it is believed that a 5-10% reduction in body weight may reduce morbidity and mortality.

Currently available prescription drugs for managing obesity generally reduce weight by decreasing dietary fat absorption, as with orlistat, or by creating an energy deficit by reducing food intake and/or increasing energy expenditure, as seen with sibutramine. The search for alternatives to presently available antiobesity agents has taken several paths one of which has focused on certain gut peptides that have been implicated in modulating satiety such as peptide YY (PYY).

PYY is a member of the pancreatic polypeptide (PP) family of hormones (along with PP and neuropeptide Y (NPY)). As with the other family members, PYY is a C-terminally amidated, 36 amino acid peptide. It is a gut endocrine peptide that was initially isolated from porcine intestine (Tatemoto and Mutt, Nature 285:417-418, 1980) and was subsequently reported to reduce high-fat food intake in rats after peripheral administration (Okada et al., Endocrinology Supplement 180, 1993) and to cause weight loss in mice after peripheral administration (Morley and Flood, Life Sciences 41:2157-2165, 1987). Multiple stored and circulating molecular forms of PYY are known to exist (Chen et al., Gastroenterology 87:1332-1338, 1984; and Roddy, et al., Regul Pept 18:201-212, 1987). One such form, PYY₃₋₃₆, was isolated from human colonic mucosal extracts (Eberlein et al., Peptides 10:797-803, 1989), and was found to be the predominant form of PYY in human postprandial plasma (Grandt et al., Regul. Pept. 51:151-159, 1994). PYY₃₋₃₆ has been reported to be a high-affinity NPY Y2 receptor (Y2R) selective agonist (Keire et al., Am. J. Physiol. Gasrointest. Liver Physiol. 279:G126-G131, 2000). Peripheral administration of PYY₃₋₃₆ has been reported to markedly reduce food intake and weight gain in rats, to decrease appetite and food intake in humans, and to decrease food intake in mice, but not in Y2R-null mice, which was said to suggest that the food intake effect requires the Y2R. In human studies, infusion of PYY₃₋₃₆ was found to significantly decrease appetite and reduce food intake by 33% over 24 hours. Infusion of PYY₃₋₃₆ to reach the normal postprandial circulatory concentrations of the peptide led to peak serum levels of PYY₃₋₃₆ within 15 minutes, followed by a rapid decline to basal levels within 30 minutes. It was reported that there was significant inhibition of food intake in the 12-hour period following the PYY₃₋₃₆ infusion, but there was essentially no effect on food intake in the 12-hour to 24-hour period. In a rat study, repeated administration of PYY₃₋₃₆ IP (injections twice daily for 7 days) reduced cumulative food intake (Batterham, et al., Nature 418:650-654, 2002).

Polypeptide-based drugs are frequently covalently attached to polymers such as polyethylene glycols to prolong their half-life *in vivo.* However, this often leads to a drastic loss of biological or pharmacological activity (Shechter et al., FEBS Letters 579:2439-2444,2005; Fuerteges and Abuchowski, J. Control Release 11:139-148, 1990; Katre, Adv. Drug Del. Sys. 10:91-114, 1993; Bailon and Berthold, Pharm. Sci. Technol. Today 1:352-356, 1996; Nucci et al., Adv. Drug Delivery Rev. 6, 1991; Delgado et al., Critical Rev. Ther. Drug Carrier Syst. 9:249-304, 1992; Fung et al., Polym. Preprints 38:565-566, 1997; Reddy, Ann. Pharmacother. 34:915-923, 2000; Veronese, Biomaterials 22:405-417, 2001). For example, Shechter et al., *supra,* reported that 40 kD pegylation of PYY₃₋₃₆ by standard chemistry, through formation of a stable bond (40 kD PEG-PYY₃₋₃₆), led to its complete inactivation in food intake studies with mice (s.c. injection). They also reported, however, that reversible pegylation of PYY₃₋₃₆ (40 kD PEG-FMS-PYY₃₋₃₆) resulted in an eight-fold increase in functional half-life (24 hrs vs. 3 hrs). See also PCT Pat. Appl. Nos. WO 2004/089279 and WO 03/026591.

### Summary of the Invention

The present invention relates to PYY agonists that are variants of PYY₃₋₃₆.

In one aspect of the present invention the PYY agonist is a variant of a mammalian PYY₃₋₃₆ in which residue 10 (glutamic acid) or residue 11 (aspartic acid) has been replaced with an amino acid "X" which is selected from the group consisting of cysteine, lysine, serine, threonine, tyrosine, and unnatural amino acids, having a functionality that is conjugatable with a hydrophilic polymer such as polyethylene glycol (PEG), e.g., a keto, thiol, hydroxyl, carboxyl, or free amino functionality, such variant being designated (E10X)PYY₃₋₃₆ or (D11X)PYY₃₋₃₆ respectively.

The residue "X" is preferably cysteine, and the corresponding variants are, therefore, (E10C)PYY₃₋₃₆ and (D11C)PYY₃₋₃₆.

In a preferred embodiment of the present invention, the PYY agonist is a variant of human PYY₃₋₃₆ (hPYY₃₋₃₆), canine PYY₃₋₃₆, feline PYY₃₋₃₆ or equine PYY₃₋₃₆, more preferably, hPYY₃₋₃₆.

In a preferred embodiment of the invention, the PYY agonist is the polypeptide (E10C)hPYY₃₋₃₆, having the amino acid sequence IKPEAPGCDASPEELNRYYASLRHYLNLVTRQRY-NH₂ [SEQ ID NO:3], or a pharmaceutically acceptable salt thereof.

In a further preferred embodiment, the PYY agonist is the polypeptide (D11C)hPYY₃₋₃₆ which has the amino acid sequence
IKPEAPGECASPEELNRYYASLRHYLNLVTRQRY-NH₂ [SEQ ID NO:4], or a pharmaceutically acceptable salt thereof.

Most preferably, the agonist is (E10C)hPYY₃₋₃₆.

The PYY agonist of the invention are preferably conjugated with a hydrophilic polymer, preferably a PEG. The agonist is preferably monopegylated, i.e., the ratio of agonist to PEG is about 1:1, which is attached at the conjugatable functionality, such as a keto, thiol, hydroxyl, carboxyl, or a free amino functionality, of "X" in (E10X)PYY₃₋₃₆ and (D11X)PYY₃₋₃₆. The PEG may be linear, branched, or pendant; more preferably, linear or branched; most preferably, linear.

In linear PEGs, one terminus of the PEG is capped by a group that is inert under the conditions of coupling the PEG to the agonist, e.g., an ether group, preferably a methoxy group. PEGs terminated in this manner (with a methoxy group) are commonly referred to as mPEGs. The other terminus is activated for coupling with the PYY agonist. Similarly, with branched PEGs useful in the present invention, all termini but one are ether-capped, and the non-ether-capped terminus is activated for coupling. In one embodiment the non-ether-capped terminus of the PEG is capped with a linker moiety ("L") linking the PEG to a functional group that is reactive with the conjugatable functionality of the amino acid X in (E10X)PYY₃₋₃₆ or (D11X)PYY₃₋₃₆ to produce a conjugate having the PEG covalently attached to the conjugatable functionality of X. In a further embodiment, the PEG is attached directly to the reactive group, without inclusion of a linker moiety. Such PEGs are frequently called "linkerless" PEGs.

For the (E10C)PYY₃₋₃₆ and (D11C)PYY₃₋₃₆ polypeptides, the non-ether capped terminus of the PEG is preferably attached to a linker linking the PEG to a maleimide or other group that will react with the thiol of the cysteine residue to produce a conjugate having the PEG covalently attached to the cysteine thiol group.

Suitable reactive PEGs for use with (E10C)hPYY₃₋₃₆ or (D11C)PYY₃₋₃₆ include PEGs of the formulas

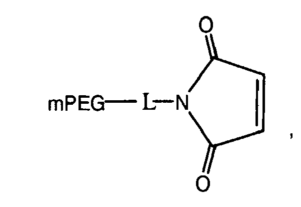

mPEG-SO₂CH=CH₂

mPEG-HN-COCH₂I

and

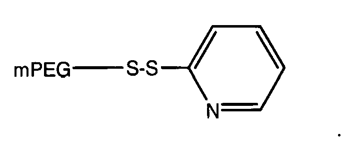

Preferably, the PEG is the mPEG maleimide depicted above which includes a linker moiety -L-, Linkerless PEG maleimides are also suitable for use in the present invention, particularly with (E10C)hPYY₃₋₃₆ or (D11C)PYY₃₋₃₆. Such linkerless PEG maleimides may be prepared as described in Goodson and Katre, Bio/Technology 8:343-346, 1990.

The conjugates produced from coupling the (E10C)hPYY₃₋₃₆ or (D11C)PYY₃₋₃₆ polypeptides with the mPEGs shown above are depicted in the following formulas, wherein -SR is the (E10C)hPYY₃₋₃₆ or (D11C)PYY₃₋₃₆ polypeptide in which the S is the sulfur atom of the cysteine thiol group:

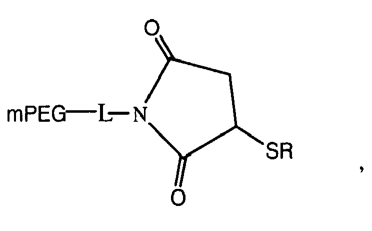

mPEG-SO₂CH₂CH₂SR

mPEG-HN-COCH₂SR and mPEG-S-SR

The linker -L- merely serves to link the PEG to the reactive functional group and is therefore not particularly limited, but, preferably, includes an alkylene group containing an ester bond, a urethane bond, an amide bond, an ether bond, a carbonate bond, or a secondary amino group.

In a preferred embodiment, particularly for linear PEGs, the linker is a group of the formula

-O(CH₂)ₚNHC(O)(CH₂)ᵣ-

in which p is an integer from 1 to 6, preferably, 1 to 3, more preferably, 2 or 3, most preferably, 3, and r is an integer from 1 to 6, preferably, 1 to 3, more preferably, 2 or 3, most preferably, 2.
A preferred linker is the group -CH₂CH₂CH₂NHCOCH₂CH₂-.

In another preferred embodiment, particularly for branched PEGs, the linker is a group of the formula

-NHC(O)(CH₂)ₛ-

in which s is an integer from 1 to 6, preferably, 1 to 3, more preferably, 2 or 3, most preferably, 2.
A preferred linker is the group -NHC(O)CH₂CH₂-.

The PEG may be linear or nonlinear, for example, branched or pendant. Preferably, the PEG is linear or branched, preferably, a linear or branched mPEG maleimide. Glycerol-branched mPEG maleimide is a preferred branched PEG. Preferably, the PEG is a linear mPEG maleimide. The PEG should have a weight-average molecular weight in the range of about 1 kD to about 50 kD. Preferably, the average molecular weight is in the range of about 5 kD to about 45 kD; more preferably, about 10-12 kD to about 40-45 kD, or about 20 kD to about 40-45 kD. Of particular interest is a linear mPEG, such as that shown in Formula 1, having a weight-average molecular weight of about 20 or about 30 kD. The glycerol-branched mPEG of Formula 2 is also of interest and, preferably, has a weight-average molecular weight of about 20 kD or about 43 kD.

Preferred PEGs, appropriately activated for conjugation with the cysteine thiol group of (E10C)hPYY₃₋₃₆ or (D11C)PYY₃₋₃₆, are the compounds of Formulas 1 and 2. In the linear mPEG of Formula 1, n is an integer in the range of about 175 to 800; preferably, about 375 to 525 or about 600 to 750, or about 425 to 475 or about 650 to 700, or about 437 to 463 or 675 to 700. In the glycerol-branched mPEG of Formula 2, each m is approximately the same and is an integer in the range of about 150 to 500; preferably, about 160 to 285 or about 400 to 525, or about 200 to 250 or 450 to 500.

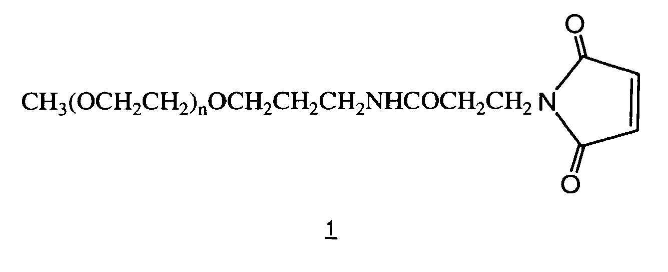

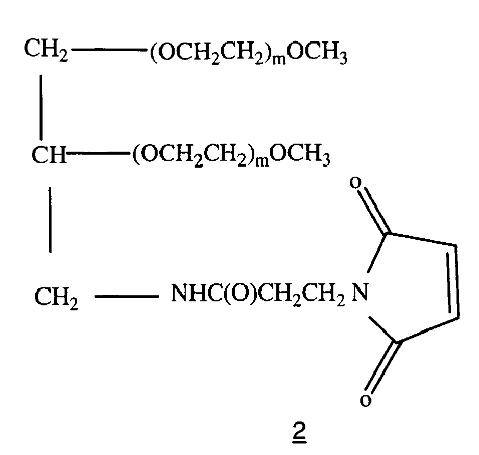

A wide variety of PEGs, appropriately activated for conjugation with target functionalities in the sidechain of peptide amino acids, e.g., keto, thiol, hydroxyl, carboxyl, or free amino functionalities, are commercially available from a number of suppliers, for example, from NOF Corporation, Tokyo, Japan, or Nektar Therapeutics Corporation, Huntsville, AL.

Another aspect of the present invention pertains to conjugates of the present PYY₃₋₃₆ variants and polyethylene glycol.

In one embodiment the conjugate is a compound of Formula 3 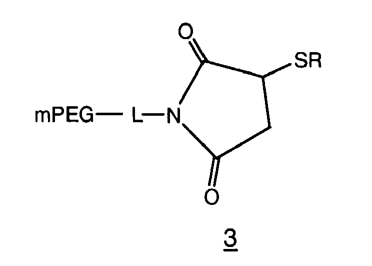 wherein the mPEG moiety is linear or branched and has a weight-average molecular weight in the range of about 1 kD to 50 kD, preferably, 5 kD to about 45 kD, more preferably, about 10 kD or 12 kD to about 40 or 45 kD, or about 20 kD to about 40 kD or 45 kD,
L is a group of the formula

-O(CH₂)ₚNHC(O)(CH₂)ᵣ-

in which p is an integer from 1 to 6; preferably, 1 to 3; more preferably, 2 or 3; most preferably, 3; (as depicted in Formula 4 below); and r is an integer from 1 to 6; preferably, 1 to 3; more preferably, 2 or 3, most preferably, 2;
or L is a group of the formula

-NHC(O)(CH₂)ₛ-

in which s is an integer from 1 to 6; preferably, 1 to 3; more preferably, 2 or 3; most preferably 2; and -SR is the polypeptide (E10C)hPYY₃₋₃₆ or (D11C)hPYY₃₋₃₆ in which S is the sulfur atom of the cysteine thiol group.

A preferred embodiment of the invention is the linear mPEG-PYY₃₋₃₆ variant conjugate of Formula 4 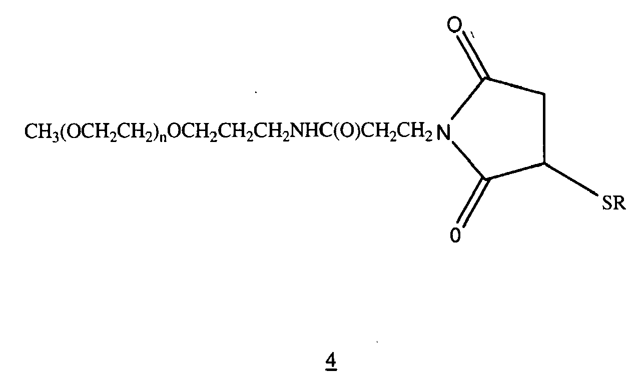 wherein n is an integer in the range of about 175 to 800; preferably, about 375 to 525 or about 600 to 750, or about 437 to 463 or about 675 to 700; and -SR is the polypeptide (E10C)hPYY₃₋₃₆ or (D11C)hPYY₃₋₃₆ in which S is the sulfur atom of the cysteine thiol group; or a pharmaceutically acceptable salt thereof. Preferably, the (CH₂CH₂O)ₙ moiety has a weight-average molecular weight of about 20 kD or 30 kD. The conjugate in which -SR is the polypeptide (E10C)hPYY₃₋₃₆ is of particular interest.

A further aspect of the invention pertains to conjugates in which the PEG moiety is branched. Preferred conjugates in this category comprise a glycerol-branched PEG moiety. Of particular interest is the conjugate of Formula 5 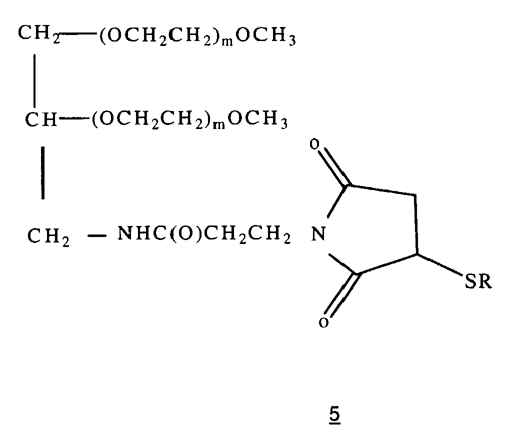 wherein each m is approximately the same and is an integer in the range of about 150 to 550; preferably, about 160 to 285 or about 400 to 525, or about 200 to 250 or about 450 to 500, and -SR is the (E10C)hPYY₃₋₃₆ or (D11C)hPYY₃₋₃₆ polypeptide in which S is the sulfur atom of the cysteine thiol group; or a pharmaceutically acceptable salt thereof. Preferably, each (CH₂CH₂O)ₘ moiety has a weight-average molecular weight in the range of about 9-11 kD or about 20-22 kD. Preferably, the combined weight-average molecular weight of the (CH₂CH₂O)ₘ moieties is about 20 kD or about 43 kD. The conjugate in which -SR is the polypeptide (E10C)hPYY₃₋₃₆ is of particular interest.

The present invention also provides a monoclonal antibody that specifically binds to a polypeptide comprising the amino acid sequence as shown in SEQ ID NO:3 or SEQ ID NO:4. In one embodiment of this aspect of the invention the polypeptide is pegylated at the cysteine residue.

In addition, the present invention provides polynucleotide sequences which encode the polypeptide sequences of the invention, preferably, they encode SEQ ID NO:3 and SEQ ID NO:4.

In another embodiment of the invention, a pharmaceutical composition is provided which comprises a PYY agonist of the present invention and a pharmaceutically acceptable carrier. In a further embodiment, the composition also comprises at least one additional pharmaceutical agent, which may be an agent useful in the treatment of the primary indication for the composition or a co-morbidity of the primary indication. The additional pharmaceutical agent is preferably an anti-obesity agent. The composition preferably comprises a therapeutically effective amount of a PYY agonist of the invention or a therapeutically effective amount of a combination of a PYY agonist of the invention and an additional pharmaceutical agent.

Also provided is a method of treating a disease, condition or disorder modulated by a Y2R agonist in mammals, which comprises peripherally administering to a mammal in need of such treatment a therapeutically effective amount of a PYY agonist of the invention. The PYY agonist of the invention may be used alone or in combination with at least one additional pharmaceutical agent that is useful in the treatment of the disease, condition or disorder or a co-morbidity of the disease, condition or disorder. Diseases, conditions, or disorders modulated by a Y2R agonist in mammals include obesity and being overweight. Co-morbidities of such diseases, conditions, or disorders would likely be incidentally improved by treatment of such diseases, conditions, or disorders. Further provided is a method of treating obesity in a mammal in need of such treatment, which comprises peripherally administering to the mammal a therapeutically effective amount of a PYY agonist of the present invention.

Also provided is a method of reducing weight or promoting weight loss (including preventing or inhibiting weight gain) in a mammal which comprises peripherally administering to the mammal a weight-controlling or weight-reducing amount of a PYY agonist of the present invention.

Also provided is a method of reducing food intake in a mammal which comprises peripherally administering to the mammal a food-intake-reducing amount of a PYY agonist of the present invention.

Also provided is a method of inducing satiety in a mammal which comprises peripherally administering to the mammal a satiety-inducing amount of a PYY agonist of the invention.

Also provided is a method of reducing caloric intake in a mammal which comprises peripherally administering to the mammal a caloric-intake-reducing amount of a PYY agonist of the invention. The PYY agonist may be administered alone or in combination with at least one additional pharmaceutical agent, preferably, an anti-obesity agent.

In each of the methods described herein and in the appendant claims, the PYY agonist may be administered alone or in combination with at least one additional pharmaceutical agent, preferably, an anti-obesity agent.

The present PYY agonists and compositions containing them are also useful in the manufacture of a medicament for the therapeutic applications mentioned herein.

### Definitions and Abbreviations

The phrase "pharmaceutically acceptable" means that the substance or composition must be compatible chemically and/or toxicologically with the other ingredients comprising a formulation, and/or the mammal being treated therewith.

The term "PYY agonist" means any compound that elicits one or more of the effects elicited by PYY, preferably PYY₃₋₃₆, *in vivo* or *in vitro*.

The phrase "therapeutically effective amount" means an amount of a PYY agonist of the present invention that reduces caloric intake, reduces body weight and/or reduces body fat with respect to appropriate control values determined prior to treatment or in a vehicle-treated group.

The term "mammal" means humans as well as all other warm-blooded members of the animal kingdom possessed of a homeostatic mechanism in the class Mammalia, e.g., companion mammals, zoo mammals and food-source mammals. Some examples of companion mammals are canines (e.g., dogs), felines (e.g., cats) and horses; some examples of food-source mammals are pigs, cattle, sheep and the like. Preferably, the mammal is a human or a companion mammal. Most preferably, the mammal is a human, male or female.

The terms "treating", "treat", or "treatment" embrace both preventative, i.e., prophylactic, and palliative treatment.

The term "peripheral administration" means administration outside of the central nervous system. Peripheral administration does not include direct administration to the brain. Peripheral administration includes, but is not limited to intravascular, intramuscular, subcutaneous, inhalation, oral, sublingual, enteral, rectal, transdermal, or intra-nasal administration.

An unnatural amino acid suitable for use in the present invention is typically any amino acid of the following formula other than the 20 naturally occurring amino acids (Cantor and Shimmel, Biophysical Chemistry, Part 1, WH Freeman & Sons, San Fransisco, 42-43, 1980), wherein R¹ is any substituent comprising a keto, thiol, carboxyl, hydroxyl or free amino functionality, such as those disclosed in U.S. Pat. Appl. Publ. No. 2005/0208536, incorporated herein by reference in its entirety. 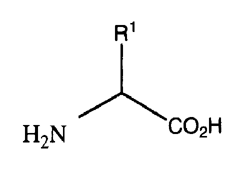 Such unnatural amino acids, for example, include thiotryosine, ornithine 3-mercaptophenylalanine, 3- or 4-aminophenylalanine, 3- or 4-acetylphenylalanine, 2- or 3-hydroxyphenylalanine (o- or m-tyrosine), hydroxymethylglycine, aminoethylglycine, 1-methyl-1-mercaptoethylglycine, aminoethylthioethylglycine and mercaptoethylglycine. Many of the unnatural amino acids useful in the present invention are commercially available. Others may be prepared by methods known in the art. For example, thiotyrosine may be prepared by the method described by Lu et al., J. Am. Chem. Soc. 119:7173-7180, 1997, incorporated herein by reference.

The term "human PYY" or "hPYY" means the 36-amino acid C-terminus amidated polypeptide having the following amino acid sequence:
YPIKPEAPGEDASPEELNRYYASLRHYLNLVTRQRY-NH₂ [SEQ ID NO:1]

The term "hPYY₃₋₃₆" means the C-terminus 34-mer hPYY having the following amino acid sequence:
IKPEAPGEDASPEELNRYYASLRHYLNLVTRQRY-NH₂ [SEQ ID NO:2]

The term "(E10C)hPYY₃₋₃₆" means the C-terminal 34-mer hPYY in which the glutamic acid residue 10 of hPYY is replaced by a cysteine residue, and which has the following amino acid sequence:
IKPEAPGCDASPEELNRYYASLRHYLNLVTRQRY-NH₂ [SEQ ID NO:3].

The term "(D11C)hPYY₃₋₃₆" means the C-terminal 34-mer hPYY in which the aspartic acid residue 11 of hPYY is replaced by a cysteine residue, and which has the following amino acid sequence:
IKPEAPGECASPEELNRYYASLRHYLNLVTRQRY-NH₂ [SEQ ID NO:4].

### Brief Description of the Drawings

Figure 1 is a reversed phase HPLC tracing of the purified (E10C)hPYY₃₋₃₆ peptide on a Zorbax Eclipse XDB-C8 column.
Figure 2 is a size exclusion HPLC tracing of the linear 30K mPEG maleimide plus (E10C)hPYY₃₋₃₆, reaction mixture on a Shodex 804 SEC column.
Figure 3 is a photo of SDS PAGE of fractions from SP Hitrap purification of linear 30K mPEG maleimide (E10C)hPYY₃₋₃₆. MW= molecular weights standards; L= column load; FT= flow through; 4-23 = elution fractions.
Figure 4 is a reversed phase HPLC tracing of the purified (D11C)hPYY₃₋₃₆ peptide on a Zorbax Eclipse XDB-C8 column.
Figure 5 is a size exclusion HPLC tracing of the linear 30K mPEG maleimide plus (D11C)hPYY₃₋₃₆ reaction mixture on a Shodex 804 SEC column.
Figure 6 is a size exclusion HPLC tracing showing the elution profile of the purified linear 30K mPEG maleimide (E10C)hPYY₃₋₃₆ product on a Shodex 804 SEC column.
Figure 7 is a size exclusion HPLC tracing showing the elution profile of the purified linear 30K mPEG maleimide (D11C)hPYY₃₋₃₆ product on a Shodex 804 SEC column.
Figure 8 is a size exclusion HPLC tracing of the glycerol-branched 43K mPEG maleimide plus (E10C)hPYY₃₋₃₆ reaction mixture on a Shodex 804 SEC column.
Figure 9 is a size exclusion HPLC tracing showing the elution profile of the purified glycerol-branched 43K mPEG maleimide (E10C)hPYY₃₋₃₆ product on a Shodex 804 SEC column.
Figure 10 is a graph of inhibition of cumulative food intake in fasted mice following intraperitoneal (IP) injection. Figure 10A shows the dose effect of native PYY₃₋₃₆ as compared to the vehicle group. Figure 10B shows the dose effect of linear 30K mPEG maleimide
   (E10C)hPYY₃₋₃₆.
Figure 11 shows the food intake effect of IP injection in fasted mice of glycerol-branched 43K mPEG maleimide(E10C)PYY₃₋₃₆ as compared to vehicle and linear 30K mPEG maleimide(E10C)PYY₃₋₃₆. Figure 11A is a line graph showing the response over 6 hours post-injection. Figure 11B is a bar graph comparing the effects over 24 hours post-injection.
Figure 12 shows the effects of IP injection of vehicle, PYY₃₋₃₆, and linear 30K mPEG maleimide(E10C)PYY₃₋₃₆ on spontaneously fed mice. Figure 12A shows the effect on food intake, and Figure 12B shows the effect on body weight.
Figure 13 shows the effects of subcutaneous (SC) injection of vehicle, PYY₃₋₃₆, and linear 30K mPEG maleimide(E10C)hPYY₃₋₃₆ on spontaneously fed mice. Figure 13A shows the effect on food intake, and Figure 13B shows the effect on body weight.
Figure 14 shows plasma exposure to PYY in mice following 0.1 mg/kg IP injection. Figure 14A demonstrates plasma PYY levels following injection of hPYY₃₋₃₆ and Figure 14B demonstrates plasma PYY levels following injection of linear 30K mPEG maleimide(E10C)hPYY₃₋₃₆.
Figure 15 is a graph of concentration response curves for PYY₃₋₃₆ or linear 30K mPEG maleimide (E10C)PYY₃₋₃₆ from the Scintillation Proximity Assay (SPA), in which the ligands compete with ¹²⁵I-PYY₁₋₃₆ for binding to the Y2R expressed on KAN-TS cells.
Figure 16 is a graph of concentration-response curves for PYY₃₋₃₆ or linear 30K mPEG maleimide (E10C)PYY₃₋₃₆ from the GTPgamma[³⁵S] Binding Assay with Y2R expressed on KAN-TS membranes.

### Detailed Description of the Invention

The present invention relates to PYY agonists that are variants of PYY₃₋₃₆ and pegylated conjugates thereof, which may have at least one improved chemical or physiological property selected from, but not limited to, decreased clearance rate, increased plasma residency duration, prolonged *in vivo* activity, increased potency, increased stability, improved solubility, and decreased antigenicity.

A preferred PYY₃₋₃₆ variant of the invention is (E10C)hPYY₃₋₃₆. Another preferred variant is (D11C)hPYY₃₋₃₆. These and other variants of the invention may be produced synthetically and by recombinant and other means, as described below and in the Examples herein or by analogous methods.

In addition to the substitutions listed above (e.g., E10C and D11C), the PYY agonists of the invention can also include one or more conservative amino acid substitutions at other amino acid positions. Conservative substitutions may be made, for example, according to the Table below. Aliphatic non-polar, polar-uncharged, and polar-charged amino acids can be substituted for another aliphatic amino acid that is non-polar, polar-uncharged, or polar-charged amino acid respectively. Preferably, such substitutions occur between amino acids in the same line of the third column of the table below. Conservative amino acid substitutions can also be made between aromatic amino acids as listed in the table below.

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

### Synthetic Production

The PYY₃₋₃₆ variants of this invention, e.g., (E10C)hPYY₃₋₃₆ and (D11C)hPYY₃₋₃₆, may be prepared using standard peptide synthesis techniques known in the art, e.g., by solid phase peptide synthesis conducted with an automatic peptide synthesizer (e.g., model 433A; Applied Biosystems, Foster City, CA) using tBoc or Fmoc chemistry. A summary of the many peptide synthesis techniques available may be found in Solid Phase Peptide Synthesis 2nd ed. (Stewart, J.M. and Young, J.D., Pierce Chemical Company, Rockford, IL, 1984). See also the book Solid-phase Organic Synthesis (Burgess, K., John Wiley & Sons, New York, NY, 2000) and the article Engels et al., Angew. Chem. Intl. Ed. 28:716- 34, 1989. All of the above references are incorporated herein by reference.

The PYY₃₋₃₆ variants of the invention are preferably conjugated with a PEG. Conjugation reactions, referred to as pegylation reactions, were historically carried out in solution with molar excess of polymer and without regard to where the polymer would attach to the protein. Such general techniques, however, have typically been proven inadequate for conjugating bioactive proteins to non-antigenic polymers while retaining sufficient bioactivity. One way to maintain the bioactivity of the PYY₃₋₃₆ agonist variant after pegylation is to substantially avoid, in the coupling process, the conjugation of any reactive groups of the variant that are associated with binding of the agonist to the target receptor. An aspect of the present invention is to provide a process of conjugating a polyethylene glycol to a PYY₃₋₃₆ variant agonist of the invention at specific reactive sites which do not interfere substantially with receptor binding site(s) in order to retain high levels of activity. Another aspect of this invention is the insertion of reactive residues into PYY₃₋₃₆ to provide the agonist variants thereof for conjugation with a polyethylene glycol with limited alteration of activity.

The chemical modification through a covalent bond may be performed under any suitable conditions generally adopted in a conjugation reaction of a biologically active substance with an activated PEG. The conjugation reaction is carried out under relatively mild conditions to avoid inactivating the PYY₃₋₃₆ variant agonist. Mild conditions include maintaining the pH of the reaction solution in the range of about 3 to 10, and the reaction temperatures in the range of about 0° to 40°C. Non-target functionalities in the PYY₃₋₃₆ variants that are reactive with the activated PEG under the pegylation conditions are preferably protected with an appropriate protecting group that is removable after pegylation at the target functionality. In pegylating free amino groups with reagents such as PEG aldehydes or PEG succinimides, a pH in the range of about 3 to 10, preferably about 4 to 7.5, is typically maintained. The coupling reaction is preferably carried out in a suitable buffer (pH 3 to 10), e.g., phosphate, MES, citrate, acetate, succinate or HEPES, for about 1 to 48 hrs at a temperature in the range of about 4° to 40°C. In pegylating thiol groups using reagents such as PEG maleimides, PEG vinyl sulfones or PEG orthopyridyl disulfides, a pH in the range of about 4 to 8 is preferably maintained. PEG amines are useful in the pegylation of keto groups, e.g., in p-acetylphenylalanine and may be prepared as described by Pillai et al., J. Org. Chem. 45:5364-5370, 1980.

The conjugation reactions of the present invention typically provide a reaction mixture or pool containing the desired mono-pegylated PYY₃₋₃₆ variant as well as unreacted PYY₃₋₃₆ variant peptide, unreacted PEG, and usually less than about 20% of high molecular weight species, which may include conjugates containing more than one PEG strand and/or aggregated species. After the unreacted species and high molecular weight species have been removed, compositions containing primarily mono-pegylated PYY₃₋₃₆ variants are recovered. Given that the conjugates often include a single polymer strand, the conjugates are substantially homogeneous.

The desired PEG-PYY₃₋₃₆ variant conjugate may be purified from the reaction mixture by conventional methods typically used for the purification of proteins, such as dialysis, salting-out, ultrafiltration, ion-exchange chromatography, hydrophobic interaction chromatography (HIC), gel chromatography and electrophoresis. Ion-exchange chromatography is particularly effective in removing any unreacted PEG or unreacted PYY₃₋₃₆ variant. Separation of the desired PEG-variant conjugate may be effected by placing the reaction mixture containing the mixed species in a buffer solution having a pH of about 4 to about 10, preferably, lower than 8 to avoid deamidation. The buffer solution preferably contains one or more buffer salts selected from, but not limited to, KCI, NaCl, K₂HPO₄, KH₂PO₄, Na₂HPO₄, NaH₂PO₄, NaHCO₃, NaBO₄ and CH₃CO₂Na.

If the buffer system used in the pegylation reaction is different from that used in the separation process, the pegylation reaction mixture is subjected to buffer exchange/diafiltration or is diluted with a sufficient amount of the initial separation buffer.

The fractionation of the conjugates into a pool containing the desired species is preferably carried out using an ion exchange chromatography medium. Such media are capable of selectively binding PEG-PYY₃₋₃₆ variant conjugates via differences in charge, which vary in a somewhat predictable fashion. For example, the surface charge of a PYY₃₋₃₆ variant is determined by the number of available charged groups on the surface of the peptide that are available for interaction with the column support uncompromised by the presence of PEG. These charged groups typically serve as the point of potential attachment of PEG polymers. Therefore, the PEG-PYY₃₋₃₆ variant conjugates will have a different charge from the other species present to allow selective isolation.

Ion exchange resins are especially preferred for purification of the present PEG-PYY₃₋₃₆ variant conjugates. Cation exchange resins such as sulfopropyl resins are used in the purification method of the present invention. A non-limiting list of cation exchange resins suitable for use with the present invention include SP-hitrap®, SP Sepharose HP® and SP Sepharose® fast flow. Other suitable cation exchange resins, e.g. S and CM resins, can also be used.

The cation exchange resin is preferably packed in a column and equilibrated by conventional means. A buffer having the same pH and osmolality as the solution of the PEG-conjugated PYY₃₋₃₆ variant is used. The elution buffer preferably contains one or more salts selected from, but not limited to, CH₃CO₂Na, HEPES, KCl, NaCl, K₂HPO₄, KH₂PO₄, Na₂HPO₄, NaH₂PO₄, NaHCO₃, NaBO₄, and (NH₄)₂CO₃. The conjugate-containing solution is then adsorbed onto the column, with unreacted PEG and some high molecular weight species not being retained. At the completion of the loading, a gradient flow of an elution buffer with increasing salt concentrations is applied to the column to elute the desired fraction of PEG-conjugated PYY₃₋₃₆ variant. The eluted, pooled fractions are preferably limited to uniform polymer conjugates after the cation exchange separation step. Any unconjugated PYY₃₋₃₆ variant species may then be washed from the column by conventional techniques. If desired, mono and multiply pegylated PYY₃₋₃₆ variant species and higher molecular weight species may be further separated from each other via additional ion exchange chromatography or size exclusion chromatography.

Techniques utilizing multiple isocratic steps of increasing concentration may be used instead of a linear gradient. Multiple isocratic elution steps of increasing concentration will result in the sequential elution of multi-pegylated/aggregated and then mono-pegylated PYY₃₋₃₆ variant conjugates. Elution techniques based on pH gradients may also be used. The temperature range for elution is generally between about 4°C and about 25°C. The elution of the PEG-PYY₃₋₃₆ variant is monitored by UV absorbance at 280 nm. Fraction collection may be achieved through simple time elution profiles.

### Recombinant Expression

### Nucleic Acid Molecules

The nucleic acid molecules encoding an (E10C)hPYY₃₋₃₆ polypeptide can comprise one of the following nucleic acid sequences (codon mutation for E10C substitution is underlined): 
atcaaacccgaggctcccggctgtgacgcctcgccggaggagctgaaccgctactacgcctccctgcgccactacctcaacctggtcacccggcagcg
gtat (SEQ ID NO: 5);
 or

atcaaacccgaggctcccggctgcgacgcctcgccggaggagctgaaccgctactacgcctccctgcgccactacctcaacctggtcacccggcagc
ggtat (SEQ ID NO: 6).

The nucleic acid molecules encoding a (D11C)hPYY₃₋₃₆ polypeptide can comprise one of the following nucleic acid sequences (codon mutation for D11C substitution is underlined): 
atcaaacccgaggctcccggcgaatgtgcctcgccggaggagctgaaccgctactacgcctccctgcgccactacctcaacctggtcacccggcagcg
gtat (SEQ ID NO: 7);
 or

atcaaacccgaggctcccggcgaatgcgcctcgccggaggagctgaaccgctactacgcctccctgcgccactacctcaacctggtcacccggcagc
ggtat (SEQ ID NO: 8).

These sequences can also include a stop codon (e.g., tga, taa, tag) at the C-terminal end, and can readily be obtained in a variety of ways including, without limitation, chemical synthesis, genetic mutation of wild type hPYY polynucleotide sequences obtained from cDNA or genomic library screening, expression library screening, and/or polymerase chain reaction (PCR) amplification of cDNA. Nucleic acid molecules encoding the (E10C)hPYY₃₋₃₆ and (D11C)hPYY₃₋₃₆ variants may be produced using site directed mutagenesis, PCR amplification, or other appropriate methods, where the primer(s) have the desired point mutations. Recombinant DNA methods and mutagenesis methods described herein are generally those set forth in Sambrook et al., Molecular Cloning: A Laboratory Manual (Cold Spring Harbor Laboratory Press, 1989) and Current Protocols in Molecular Biology (Ausubel et al., eds., Green Publishers Inc. and Wiley and Sons 1994). Should it be desired that another non-naturally-occurring amino acid is substituted for E10 or D11, such a peptide can be recombinantly expressed using methods as disclosed in, for example, U.S. Pat. Appl. Publ. No. 2005/0208536, incorporated herein by reference.

Nucleic acid polynucleotides encoding the amino acid sequence of hPYYs may be identified by expression cloning which employs the detection of positive clones based upon a property of the expressed protein. Typically, nucleic acid libraries are screened by the binding of an antibody or other binding partner (e.g., receptor or ligand) to cloned proteins that are expressed and displayed on a host cell surface. The antibody or binding partner is modified with a detectable label to identify those cells expressing the desired clone.

Recombinant expression techniques conducted in accordance with the descriptions set forth below may be followed to produce the (E10C)hPYY₃₋₃₆ and (D11C)hPYY₃₋₃₆ encoding polynucleotides and to express the encoded polypeptides. For example, by inserting a nucleic acid sequence that encodes the amino acid sequence of an (E10C)hPYY₃₋₃₆ or a (D11C)hPYY₃₋₃₆ variant into an appropriate vector, one skilled in the art can readily produce large quantities of the desired nucleotide sequence. The sequences can then be used to generate detection probes or amplification primers. Alternatively, a polynucleotide encoding the amino acid sequence of an (E10C)hPYY₃₋₃₆ or a (D11C)hPYY₃₋₃₆ polypeptide can be inserted into an expression vector. By introducing the expression vector into an appropriate host, the encoded (E10C)hPYY₃₋₃₆ or (D11C)hPYY₃₋₃₆ variant may be produced in large amounts.

Another method for obtaining a suitable nucleic acid sequence is the polymerase chain reaction (PCR). In this method, cDNA is prepared from poly(A)+RNA or total RNA using the enzyme reverse transcriptase. Two primers, typically complementary to two separate regions of cDNA encoding the amino acid sequence of an (E10C)hPYY₃₋₃₆ or a (D11C)hPYY₃₋₃₆ variant, are then added to the cDNA along with a polymerase such as Taq polymerase, and the polymerase amplifies the cDNA region between the two primers.

Another means of preparing a nucleic acid molecule encoding the amino acid sequence of an (E10C)hPYY₃₋₃₆ or a (D11C)hPYY₃₋₃₆ variant is chemical synthesis using methods well known to the skilled artisan such as those described by Engels et al., Angew. Chem. Intl. Ed. 28:716- 34, 1989. These methods include the phosphotriester, phosphoramidite, and H-phosphonate methods for nucleic acid synthesis. A preferred method for such chemical synthesis is polymer-supported synthesis using standard phosphoramidite chemistry. Typically, the DNA encoding the amino acid sequence of an (E10C)hPYY₃₋₃₆ will be about one hundred nucleotides in length. Nucleic acids larger than about 100 nucleotides can be synthesized as several fragments using these methods. The fragments can then be ligated together to form the full-length nucleotide sequence of an (E10C)hPYY₃₋₃₆ gene.

The DNA fragment encoding the amino-terminus of the polypeptide can have an ATG, which encodes a methionine residue. This methionine may or may not be present on the mature form of the (E10C)hPYY₃₋₃₆ or (DIIC) LP443-36, depending on whether the polypeptide produced in the host cell is designed to be secreted from that cell. The codon encoding isoleucine can also be used as a start site. Other methods known to the skilled artisan may be used as well. In certain embodiments, nucleic acid variants contain codons which have been altered for optimal expression of an (E10C)hPYY₃₋₃₆ or a (D11C)hPYY₃₋₃₆ in a given host cell. Particular codon alterations will depend upon the (E10C)hPYY₃₋₃₆ or (D11C)hPYY₃₋₃₆ and the host cell selected for expression. Such "codon optimization" can be carried out by a variety of methods, for example, by selecting codons which are preferred for use in highly expressed genes in a given host cell. Computer algorithms which incorporate codon frequency tables such as "Eco_high.Cod" for codon preference of highly expressed bacterial genes may be used and are provided by the University of Wisconsin Package Version 9.0 (Genetics Computer Group, Madison, Wis.). Other useful codon frequency tables include "Celegans_high.cod," "Celegans_low.cod," "Drosophila_high.cod," "Human_high.cod," "Maize_high.cod," and "Yeast_high.cod."

### Vectors and Host Cells

A nucleic acid molecule encoding the amino acid sequence of an (E10C)hPYY₃₋₃₆ or a (D11C)hPYY₃₋₃₆ is inserted into an appropriate expression vector using standard ligation techniques. The vector is typically selected to be functional in the particular host cell employed (i.e., the vector is compatible with the host cell machinery such that amplification of the gene and/or expression of the gene can occur). A nucleic acid molecule encoding the amino acid sequence of an (E10C)hPYY₃₋₃₆ or a (D11C)hPYY₃₋₃₆ may be amplified/expressed in prokaryotic, yeast, insect (baculovirus systems) and/or eukaryotic host cells. For a review of expression vectors, see Meth. Enz., vol. 185 (D. V. Goeddel, ed., Academic Press, 1990).

Typically, expression vectors used in any of the host cells will contain sequences for plasmid maintenance and for cloning and expression of exogenous nucleotide sequences. Such sequences, collectively referred to as "flanking sequences" in certain embodiments, will typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Each of these sequences is discussed below.

Optionally, the vector may contain a "tag"-encoding sequence, i.e., an oligonucleotide molecule located at the 5' or 3' end of the (E10C)hPYY₃₋₃₆ or the (D11C)hPYY₃₋₃₆ coding sequence; the oligonucleotide sequence encodes polyHis (such as hexaHis), or another "tag" such as FLAG, HA (hemaglutinin influenza virus), or myc for which commercially available antibodies exist. This tag is typically fused to the polypeptide upon expression of the polypeptide, and can serve as a means for affinity purification of the (E10C)hPYY₃₋₃₆ or the (D11C)hPYY₃₋₃₆ from the host cell. Affinity purification can be accomplished, for example, by column chromatography using antibodies against the tag as an affinity matrix. Optionally, the tag can subsequently be removed from the purified (E10C)hPYY₃₋₃₆ or (D11C)hPYY₃₋₃₆ by various means such as using certain peptidases for cleavage, e.g., enterokinase digestion 3' of a FLAG tag sequence that is upstream of the one of the amino acid sequences as shown in SEQ ID NOs: 3-4.

Flanking sequences may be homologous (i.e., from the same species and/or strain as the host cell), heterologous (i.e., from a species other than the host cell species or strain), hybrid (i.e., a combination of flanking sequences from more than one source), or synthetic, or the flanking sequences may be native sequences which normally function to regulate hPYY₃₋₃₆ expression. The source of a flanking sequence may be any prokaryotic or eukaryotic organism, any vertebrate or invertebrate organism, or any plant, provided that the flanking sequence is functional in, and can be activated by, the host cell machinery.

Useful flanking sequences may be obtained by any of several methods well known in the art. Typically, flanking sequences useful herein, other than the PYY gene flanking sequences, will have been previously identified by mapping and/or by restriction endonuclease digestion and can thus be isolated from the proper tissue source using the appropriate restriction endonucleases. In some cases, the full nucleotide sequence of a flanking sequence may be known. Here, the flanking sequence may be synthesized using the methods described herein for nucleic acid synthesis or cloning.

Where all or only a portion of the flanking sequence is known, it may be obtained using PCR and/or by screening a genomic library with a suitable oligonucleotide and/or flanking sequence fragment from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen® column chromatography (Chatsworth, CA), or other methods known to the skilled artisan. The selection of suitable enzymes to accomplish this purpose will be readily apparent to one of skill in the art.

An origin of replication is typically a part of those prokaryotic expression vectors purchased commercially, and the origin aids in the amplification of the vector in a host cell. Amplification of the vector to a certain copy number can, in some cases, be important for the optimal expression of an (E10C)hPYY₃₋₃₆ or a (D11C)hPYY₃₋₃₆. If the vector of choice does not contain an origin of replication site, one may be chemically synthesized based on a known sequence, and ligated into the vector. For example, the origin of replication from the plasmid pBR322 (New England Biolabs, Beverly, MA) is suitable for most gram-negative bacteria and various origins (e.g., SV40, polyoma, adenovirus, vesicular stomatitus virus (VSV), or papillomaviruses such as HPV or BPV) are useful for cloning vectors in mammalian cells. Generally, the origin of replication component is not needed for mammalian expression vectors (for example, the SV40 origin is often used only because it contains the early promoter).

A transcription termination sequence is typically located 3' of the end of a polypeptide coding region and serves to terminate transcription. Usually, a transcription termination sequence in prokaryotic cells is a G-C rich fragment followed by a poly-T sequence. While the sequence is easily cloned from a library or even purchased commercially as part of a vector, it can also be readily synthesized using methods for nucleic acid synthesis such as those described herein.

A selectable marker gene element encodes a protein necessary for the survival and growth of a host cell grown in a selective culture medium. Typical selection marker genes encode proteins that (a) confer resistance to antibiotics or other toxins, e.g., ampicillin, tetracycline, or kanamycin for prokaryotic host cells; (b) complement auxotrophic deficiencies of the cell; or (c) supply critical nutrients not available from complex media. Preferred selectable markers are the kanamycin resistance gene, the ampicillin resistance gene, and the tetracycline resistance gene. A neomycin resistance gene may also be used for selection in prokaryotic and eukaryotic host cells.

Other selection genes may be used to amplify the gene that will be expressed. Amplification is the process wherein genes that are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The mammalian cell transformants are placed under selection pressure wherein only the transformants are uniquely adapted to survive by virtue of the selection gene present in the vector. Selection pressure is imposed by culturing the transformed cells under conditions in which the concentration of selection agent in the medium is successively changed, thereby leading to the amplification of both the selection gene and the DNA that encodes an (E10C)hPYY₃₋₃₆ or (D11C)hPYY₃₋₃₆. As a result, increased quantities of (E10C)hPYY₃₋₃₆ or (D11C)hPYY₃₋₃₆ are synthesized from the amplified DNA.

A ribosome binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of an (E10C)hPYY₃₋₃₆ or a (D11C)hPYY₃₋₃₆ to be expressed. The Shine-Dalgarno sequence is varied but is typically a polypurine (i.e., having a high A-G content). Many Shine-Dalgarno sequences have been identified, each of which can be readily synthesized using methods set forth herein and used in a prokaryotic vector.

A leader, or signal, sequence may be used to direct an (E10C)hPYY₃₋₃₆ or (D11C)hPYY₃₋₃₆ out of the host cell. Typically, a nucleotide sequence encoding the signal sequence is positioned in the coding region of the (E10C)hPYY₃₋₃₆ or the (D11C)hPYY₃₋₃₆ nucleic acid molecule, or directly at the 5' end of an (E10C)hPYY₃₋₃₆ or a (D11C)hPYY₃₋₃₆ coding region. Many signal sequences have been identified, and any of those that are functional in the selected host cell may be used in conjunction with an (E10C)hPYY₃₋₃₆ or (D11C)hPYY₃₋₃₆ nucleic acid molecule. Therefore, a signal sequence may be homologous (naturally occurring) or heterologous to the (E10C)hPYY₃₋₃₆ or (D11C)hPYY₃₋₃₆ nucleic acid molecule. Additionally, a signal sequence may be chemically synthesized using methods described herein. In most cases, the secretion of an (E10C)hPYY₃₋₃₆ or a (D11C)hPYY₃₋₃₆ from the host cell via the presence of a signal peptide will result in the removal of the signal peptide from the secreted (E10C)hPYY₃₋₃₆ or (D11C)hPYY₃₋₃₆. The signal sequence may be a component of the vector, or it may be a part of an (E10C)hPYY₃₋₃₆ or a (D11C)hPYY₃₋₃₆ nucleic acid molecule that is inserted into the vector.

A nucleotide sequence encoding a native hPYY₃₋₃₆ signal sequence may be joined to an (E10C)hPYY₃₋₃₆ or a (D11C)hPYY₃₋₃₆ coding region or a nucleotide sequence encoding a heterologous signal sequence may be joined to an (E10C)hPYY₃₋₃₆ or a (D11C)hPYY₃₋₃₆ coding region. The heterologous signal sequence selected should be one that is recognized and processed, i.e., cleaved by a signal peptidase, by the host cell. For prokaryotic host cells that do not recognize and process the native hPYY signal sequence, the signal sequence is substituted by a prokaryotic signal sequence selected, for example, from the group of the alkaline phosphatase, penicillinase, or heat-stable enterotoxin II leaders. For yeast secretion, the native hPYY signal sequence may be substituted by the yeast invertase, alpha factor, or acid phosphatase leaders. In mammalian cell expression, the native signal sequence is satisfactory, although other mammalian signal sequences may be suitable.

In many cases, transcription of a nucleic acid molecule is increased by the presence of one or more introns in the vector; this is particularly true where a polypeptide is produced in eukaryotic host cells, especially mammalian host cells. The introns used may be naturally occurring within the hPYY gene especially where the gene used is a full-length genomic sequence or a fragment thereof. Where the intron is not naturally occurring within the gene (as for most cDNAs), the intron may be obtained from another source. The position of the intron with respect to flanking sequences and the hPYY gene is generally important, as the intron must be transcribed to be effective. Thus, when an (E10C)hPYY₃₋₃₆ or a (D11C)hPYY₃₋₃₆ encoding cDNA molecule is being transcribed, the preferred position for the intron is 3' to the transcription start site and 5' to the poly-A transcription termination sequence. Preferably, the intron or introns will be located on one side or the other (i.e., 5' or 3') of the cDNA such that it does not interrupt the coding sequence. Any intron from any source, including viral, prokaryotic and eukaryotic (plant or animal) organisms, may be used, provided that it is compatible with the host cell into which it is inserted. Also included herein are synthetic introns. Optionally, more than one intron may be used in the vector.

Expression and cloning vectors will typically contain a promoter that is recognized by the host organism and operably linked to the molecule encoding the (E10C)hPYY₃₋₃₆ or (D11C)hPYY₃₋₃₆. Promoters are untranscribed sequences located upstream (i.e., 5') to the start codon of a structural gene (generally within about 100 to 1000 bp) that control the transcription of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no control over gene expression. A large number of promoters, recognized by a variety of potential host cells, are well known. A suitable promoter is operably linked to the DNA encoding (E10C)hPYY₃₋₃₆ or (D11C)hPYY₃₋₃₆ by removing the promoter from the source DNA by restriction enzyme digestion and inserting the desired promoter sequence into the vector. The native hPYY₃₋₃₆ promoter sequence may be used to direct amplification and/or expression of an (E10C)hPYY₃₋₃₆ or (D11C)hPYY₃₋₃₆ nucleic acid molecule. However, a heterologous promoter is preferred, if it permits greater transcription and higher yields of the expressed protein as compared to the native promoter, and if it is compatible with the host cell system that has been selected for use.

Promoters suitable for use with prokaryotic hosts include the beta-lactamase and lactose promoter systems; *E. coli* T7 inducible RNA polymerase; alkaline phosphatase; a tryptophan (trp) promoter system; and hybrid promoters such as the tac promoter. Other known bacterial promoters are also suitable. Their sequences have been published, thereby enabling one skilled in the art to ligate them to the desired DNA sequence, using linkers or adapters as needed to supply any useful restriction sites.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowlpox virus, adenovirus (such as Adenovirus 2), bovine papilloma virus, avian sarcoma virus, cytomegalovirus, retroviruses, hepatitis-B virus and most preferably Simian Virus 40 (SV40). Other suitable mammalian promoters include heterologous mammalian promoters, for example, heat-shock promoters and the actin promoter.

Additional promoters which may be of interest in controlling expression of (E10C)hPYY₃₋₃₆ or (D11C)hPYY₃₋₃₆ include, but are not limited to: the SV40 early promoter region (Bemoist and Chambon, Nature 290:304-10, 1981); the CMV promoter; the promoter contained in the 3' long terminal repeat of Rous sarcoma virus (Yamamoto et al, Cell 22:787- 97, 1980); the herpes thymidine kinase promoter (Wagner et al., Proc. Natl. Acad. Sci. U.S.A. 78:1444-45, 1981); the regulatory sequences of the metallothionine gene (Brinster et al., Nature 296:39-42, 1982); prokaryotic expression vectors such as the beta- lactamase promoter (Villa-Kamaroff et al., Proc. Natl. Acad. Sci. U.S.A. 75:3727-31, 1978); or the tac promoter (DeBoer et al., Proc. Natl. Acad. Sci. U.S.A., 80:21-25, 1983).

An enhancer sequence may be inserted into the vector to increase the transcription in higher eukaryotes of a DNA encoding an (E10C)hPYY₃₋₃₆ or (D11C)hPYY₃₋₃₆. Enhancers are cis-acting elements of DNA, usually about 10- 300 bp in length, that act on the promoter to increase transcription. Enhancers are relatively orientation and position independent. They have been found 5' and 3' to the transcription unit. Several enhancer sequences available from mammalian genes are known (e.g., globin, elastase, albumin, alpha- feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters. While an enhancer may be spliced into the vector at a position 5' or 3' to an (E10C)hPYY₃₋₃₆ or (D11C)hPYY₃₋₃₆ encoding nucleic acid molecule, it is typically located at a site 5' to the promoter.

Expression vectors may be constructed from a starting vector such as a commercially available vector. Such vectors may or may not contain all of the desired flanking sequences. Where one or more of the flanking sequences described herein are not already present in the vector, they may be individually obtained and ligated into the vector. Methods used for obtaining each of the flanking sequences are well known to one skilled in the art.

Preferred vectors are those which are compatible with bacterial, insect, and mammalian host cells. Such vectors include, inter alia, pCRII, pCR3, and pcDNA3.1 (Invitrogen, Carlsbad, CA), pBSII (Stratagene, La Jolla, CA), pET15 (Novagen, Madison, WI), pGEX (Pharmacia Biotech, Piscataway, NJ), pEGFP-N2 (Clontech, Palo Alto, CA), pETL (BlueBacll, Invitrogen), pDSR-alpha (PCT Appl. Publ. No. WO 90/14363) and pFastBacDual (Gibco-BRL, Grand Island, NY).

Additional suitable vectors include, but are not limited to, cosmids, plasmids, or modified viruses, but it will be appreciated that the vector system must be compatible with the selected host cell. Such vectors include, but are not limited to, plasmids such as Bluescript^{®} plasmid derivatives (a high copy number ColE1-based phagemid, Stratagene), PCR cloning plasmids designed for cloning Taq- amplified PCR products (e.g., TOPO^{®} TA Cloning^{®} Kit, PCR2.1^{®} plasmid derivatives, Invitrogen), and mammalian, yeast or virus vectors such as a baculovirus expression system (pBacPAK plasmid derivatives, Clontech).

After the vector has been constructed and a nucleic acid molecule encoding an (E10C)hPYY₃₋₃₆ or (D11C)hPYY₃₋₃₆ polypeptide has been inserted into the proper site of the vector, the completed vector may be inserted into a suitable host cell for amplification and/or polypeptide expression. The transformation of an expression vector for an (E10C)hPYY₃₋₃₆ or (D11C)hPYY₃₋₃₆ polypeptide into a selected host cell may be accomplished by well known methods including methods such as transfection, infection, electroporation, microinjection, lipofection, DEAE-dextran method, or other known techniques. The method selected will in part be a function of the type of host cell to be used. These methods and other suitable methods are well known to the skilled artisan, and are set forth, for example, in Sambrook et al., *supra.*

Host cells may be prokaryotic host cells (such as *E*. *coli*) or eukaryotic host cells (such as a yeast, insect, or vertebrate cell). The host cell, when cultured under appropriate conditions, synthesizes an (E10C)hPYY₃₋₃₆ or (D11C)hPYY₃₋₃₆ polypeptide which can subsequently be collected from the culture medium (if the host cell secretes it into the medium) or directly from the host cell producing it (if it is not secreted). The selection of an appropriate host cell will depend upon various factors, such as desired expression levels, polypeptide modifications that are desirable or necessary for activity (such as glycosylation or phosphorylation) and ease of folding into a biologically active molecule.

A number of suitable host cells are known in the art and many are available from the American Type Culture Collection (ATCC), Manassas, Va. Examples include, but are not limited to, mammalian cells, such as Chinese hamster ovary cells (CHO), CHO DHFR(-) cells (Urlaub et al., Proc. Natl. Acad. Sci. U.S.A. 97:4216-20, 1980), human embryonic kidney (HEK) 293 or 293T cells, or 3T3 cells. The selection of suitable mammalian host cells and methods for transformation, culture, amplification, screening, product production, and purification are known in the art. Other suitable mammalian cell lines are monkey COS-1 and COS-7 cell lines, and the CV-1 cell line. Further exemplary mammalian host cells include primate cell lines and rodent cell lines, including transformed cell lines. Normal diploid cells, cell strains derived from *in vitro* culture of primary tissue, as well as primary explants, are also suitable. Candidate cells may be genotypically deficient in the selection gene, or may contain a dominantly acting selection gene. Other suitable mammalian cell lines include, but are not limited to, mouse neuroblastoma N2A cells, HeLa, mouse L-929 cells, 3T3 lines derived from Swiss, Balb-c or NIH mice, BHK or HaK hamster cell lines. Each of these cell lines is known by and available to those skilled in the art of protein expression.

Similarly useful as suitable host cells are bacterial cells. For example, the various strains of *E*. *coli* (e.g., HB101, DH5α, DH10, and MC1061) are well known as host cells in the field of biotechnology. Various strains of *B. subtilis, Pseudomonas* spp., other *Bacillus* spp., and *Streptomyces* spp. may also be employed in this method.

Many strains of yeast cells known to those skilled in the art are also available as host cells for the expression of (E10C)hPYY₃₋₃₆ and (D11C)hPYY₃₋₃₆ polypeptides. Preferred yeast cells include, for example, *Saccharomyces cerivisae* and *Pichia pastoris*.

Additionally, where desired, insect cell systems may be utilized for the expression of (E10C)hPYY₃₋₃₆ and (D11C)hPYY₃₋₃₆. Such systems are described, for example, in Kitts et al., 1993, Biotechniques 14:810-17; Lucklow, Curr. Opin. Biotechnol. 4:564-72, 1993; and Lucklow et al., J. Virol., 67:4566-79, 1993. Preferred insect cells are Sf-9 and Hi5 (Invitrogen).

### (E10C)hPYY₃₋₃₆ and (D11C)hPYY₃₋₃₆Polypeptide Production

A host cell line comprising an (E10C)hPYY₃₋₃₆ or (D11C)hPYY₃₋₃₆ expression vector may be cultured using standard media well known to the skilled artisan. The media will usually contain all nutrients necessary for the growth and survival of the cells. Suitable media for culturing *E*. *coli* cells include, for example, Luria Broth (LB) and/or Terrific Broth (TB). Suitable media for culturing eukaryotic cells include Roswell Park Memorial Institute medium 1640 (RPMI 1640), Minimal Essential Medium (MEM) and/or Dulbecco's Modified Eagle Medium (DMEM), all of which may be supplemented with serum and/or growth factors as necessary for the particular cell line being cultured. A suitable medium for insect cultures is Grace's medium supplemented with yeastolate, lactalbumin hydrolysate, and/or fetal calf serum, as necessary.

Typically, an antibiotic or other compound useful for selective growth of transfected or transformed cells is added as a supplement to the media. The compound to be used will be dictated by the selectable marker element present on the plasmid with which the host cell was transformed. For example, where the selectable marker element is kanamycin resistance, the compound added to the culture medium will be kanamycin. Other compounds for selective growth include ampicillin, tetracycline, and neomycin.

The amount of an (E10C)hPYY₃₋₃₆ or (D11C)hPYY₃₋₃₆ polypeptide produced by a host cell can be evaluated using standard methods known in the art. Such methods include, without limitation, Western blot analysis, SDS-polyacrylamide gel electrophoresis, non-denaturing gel electrophoresis, High Performance Liquid Chromatography (HPLC) separation, immunoprecipitation, and/or activity assays such as DNA binding gel shift assays.

If an (E10C)hPYY₃₋₃₆ or (D11C)hPYY₃₋₃₆ has been designed to be secreted from the host cell line, the majority of polypeptide may be found in the cell culture medium. If, however, the polypeptide is not secreted from the host cells, it will be present in the cytoplasm and/or the nucleus (for eukaryotic host cells) or in the cytosol (for gram-negative bacteria host cells).

For an (E10C)hPYY₃₋₃₆ or (D11C)hPYY₃₋₃₆ situated in the host cell cytoplasm and/or nucleus (for eukaryotic host cells) or in the cytosol (for bacterial host cells), the intracellular material (including inclusion bodies for gram-negative bacteria) can be extracted from the host cell using any standard technique known to the skilled artisan. For example, the host cells can be lysed to release the contents of the periplasm/cytoplasm by French press, homogenization, and/or sonication, followed by centrifugation.

If an (E10C)hPYY₃₋₃₆ or (D11C)hPYY₃₋₃₆ has formed inclusion bodies in the cytosol, the inclusion bodies can often bind to the inner and/or outer cellular membranes and thus will be found primarily in the pellet material after centrifugation. The pellet material can then be treated at pH extremes or with a chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. The solubilized (E10C)hPYY₃₋₃₆ or (D11C)hPYY₃₋₃₆ can then be analyzed using gel electrophoresis, immunoprecipitation, or the like. If it is desired to isolate the polypeptide, isolation may be accomplished using standard methods such as those described herein and in Marston et al., Meth. Enz. 182:264-75, 1990.

If inclusion bodies are not formed to a significant degree upon expression of an (E10C)hPYY₃₋₃₆ or a (D11C)hPYY₃₋₃₆, then the polypeptide will be found primarily in the supernatant after centrifugation of the cell homogenate. The polypeptide may be further isolated from the supernatant using methods such as those described herein.

The purification of an (E10C)hPYY₃₋₃₆ or (D11C)hPYY₃₋₃₆ from solution can be accomplished using a variety of techniques. If the polypeptide has been synthesized such that it contains a tag such as Hexahistidine 9 or other small peptide such as FLAG (Eastman Kodak Co., New Haven, CT) or myc (Invitrogen) at either its carboxyl or amino-terminus, it may be purified in a one-step process by passing the solution through an affinity column where the column matrix has a high affinity for the tag.

For example, polyhistidine binds with great affinity and specificity to nickel. Thus, an affinity column of nickel (such as the Qiagen® nickel columns) can be used for purification. See, e. g., Current Protocols in Molecular Biology § 10.11.8 (Ausubel et al., eds., Green Publishers Inc. and Wiley and Sons, 1993).

Additionally, an (E10C)hPYY₃₋₃₆ or a (D11C)hPYY₃₋₃₆ polypeptide may be purified through the use of a monoclonal antibody that is capable of specifically recognizing and binding to an (E10C)hPYY₃₋₃₆ or (D11C)hPYY₃₋₃₆ polypeptide.

In situations where it is preferable to partially or completely purify an (E10C)hPYY₃₋₃₆ or (D11C)hPYY₃₋₃₆ polypeptide such that it is partially or substantially free of contaminants, standard methods known to those skilled in the art may be used. Such methods include, without limitation, separation by electrophoresis followed by electroelution, various types of chromatography (affinity, immunoaffinity, molecular sieve, and ion exchange), HPLC, and preparative isoelectric focusing ("Isoprime" machine/technique, Hoefer Scientific, San Francisco, CA). In some cases, two or more purification techniques may be combined to achieve increased purity.

A number of additional methods for producing polypeptides are known in the art, and the methods can be used to produce (E10C)hPYY₃₋₃₆ and (D11C)hPYY₃₋₃₆ polypeptides. See, e.g., Roberts et al., Proc. Natl. Acad. Sci. U.S.A. 94:12297-303, 1997, which describes the production of fusion proteins between an mRNA and its encoded peptide. See also, Roberts, Curr. Opin. Chem. Biol. 3:268-73, 1999.

Processes for producing peptides or polypeptides are also described in U.S. Pat. Nos.: 5,763,192; 5,814,476; 5,723,323; and 5,817,483. The process involves producing stochastic genes or fragments thereof, and then introducing these genes into host cells which produce one or more proteins encoded by the stochastic genes. The host cells are then screened to identify those clones producing peptides or polypeptides having the desired activity. Other processes for recombinant peptide expression are disclosed in U.S. Pat. Nos.: 6,103,495, 6,210,925, 6,627,438, and 6,737,250. The process utilizes *E*. *coli* and the *E*. *coli* general secretory pathway. The peptide is fused to a signal sequence; thus, the peptide is targeted for secretion.

Another method for producing peptides or polypeptides is described in PCT Pat. Appl. Publ. No. WO 99/15650. The published process, termed random activation of gene expression for gene discovery, involves the activation of endogenous gene expression or over- expression of a gene by *in situ* recombination methods. For example, expression of an endogenous gene is activated or increased by integrating a regulatory sequence into the target cell which is capable of activating expression of the gene by non-homologous or illegitimate recombination. The target DNA is first subjected to radiation, and a genetic promoter inserted. The promoter eventually locates a break at the front of a gene, initiating transcription of the gene. This results in expression of the desired peptide or polypeptide.

### Amidation

Amidation of a peptide, produced either synthetically or recombinantly, is accomplished by an enzyme called peptidyl-glycine alpha-amidating monooxygenase (PAM). When producing (E10C)hPYY₃₋₃₆ or (D11C)hPYY₃₋₃₆ peptides recombinantly using a bacterial expression system, the peptides can be C-terminal amidated by an *in vitro* reaction using recombinant PAM enzyme. The PAM enzyme source, methods of its production and purification, and methods that can be used to amidate (E10C)hPYY₃₋₃₆ or (D11C)hPYY₃₋₃₆ peptides are described, for example, in U.S. Pat. Nos.: 4,708,934, 5,789,234, and 6,319,685.

### Selective (E10C)hPYY₃₋₃₆ and (D11C)hPYY₃₋₃₆ Antibodies

Antibodies and antibody fragments that specifically bind (E10C)hPYY₃₋₃₆ or (D11C)hPYY₃₋₃₆ polypeptides, with or without pegylation at the site of cysteine substitution (as described herein), but do not selectively bind native hPYY₃₋₃₆, are within the scope of the present invention. The antibodies may be polyclonal, including monospecific polyclonal; monoclonal; recombinant; chimeric; humanized, such as CDR-grafted; human; single chain; and/or bispecific; as well as fragments; variants; or derivatives thereof. Antibody fragments include those portions of the antibody that bind to an epitope on the (E10C)hPYY₃₋₃₆ or (D11C)hPYY₃₋₃₆ polypeptide. Examples of such fragments include Fab and F(ab') fragments generated by enzymatic cleavage of full-length antibodies. Other binding fragments include those generated by recombinant DNA techniques, such as the expression of recombinant plasmids containing nucleic acid sequences encoding antibody variable regions.

Polyclonal antibodies directed toward an (E10C)hPYY₃₋₃₆ or (D11C)hPYY₃₋₃₆ polypeptide generally are produced in animals (e.g., rabbits or mice) by means of multiple SC or IP injections of (E10C)hPYY₃₋₃₆ or (D11C)hPYY₃₋₃₆ polypeptide and an adjuvant. It may be useful to conjugate an (E10C)hPYY₃₋₃₆ or a (D11C)hPYY₃₋₃₆ polypeptide to a carrier protein that is immunogenic in the species to be immunized, such as keyhole limpet hemocyanin, serum albumin, bovine thyroglobulin, or soybean trypsin inhibitor. Also, aggregating agents such as alum are used to enhance the immune response. After immunization, the animals are bled and the serum is assayed for anti-(E10C)hPYY₃₋₃₆ or anti-(D11C)hPYY₃₋₃₆ antibody titer.

Monoclonal antibodies directed toward (E10C)hPYY₃₋₃₆ or (D11C)hPYY₃₋₃₆ polypeptides are produced using any method that provides for the production of antibody molecules by continuous cell lines in culture. Examples of suitable methods for preparing monoclonal antibodies include the hybridoma methods of Kohler et al., Nature 256:495-97, 1975, and the human B-cell hybridoma method (Kozbor, J. Immunol. 133:3001, 1984; Brodeur et al., Monoclonal Antibody Production Techniques and Applications, 51-63 (Marcel Dekker, Inc., 1987). Also provided by the invention are hybridoma cell lines that produce monoclonal antibodies reactive with (E10C)hPYY₃₋₃₆ or (D11C)hPYY₃₋₃₆ polypeptides.

Preferred methods for determining monoclonal antibody specificity and affinity by competitive inhibition can be found in Harlow and Lane, Antibodies: A Laboratory Manual (Cold Spring Harbor Laboratories, 1989); Current Protocols in Immunology (Colligan et al., eds., Greene Publishing Assoc. and Wiley Interscience, 1993); and Muller, Meth. Enzymol. 92:589-601, 1988.

Chimeric antibodies of the present invention may comprise individual H and/or L immunoglobulin chains. A preferred chimeric H chain comprises an antigen-binding region derived from the H chain of a non-human antibody specific for an (E10C)hPYY₃₋₃₆ or (D11C)hPYY₃₋₃₆ polypeptide which is linked to at least a portion of a human H chain C region (C_{H}), such as CH₁ or CH₂. A preferred chimeric L chain comprises an antigen-binding region derived from the L chain of a non- human antibody specific for an (E10C)hPYY₃₋₃₆ or (D11C)hPYY₃₋₃₆ polypeptide which is linked to at least a portion of a human L chain C region (C_{L}). Chimeric antibodies and methods for their production are known in the art. See Cabilly et al., Proc. Natl. Acad. Sci. U.S.A. 81:3273-77, 1984; Morrison et al., Proc. Natl. Acad. Sci. U. S.A. 81:6851-55, 1984; Boulianne et al., Nature 312:643-46, 1984; Neuberger et al., Nature 314:268- 70, 1985; Liu et al., Proc. Natl. Acad. Sci. U.S.A. 84:3439-43, 1987; and Harlow and Lane, *supra.*

Selective binding agents having chimeric H chains and L chains of the same or different variable region binding specificity can also be prepared by appropriate association of the individual polypeptide chains, according to methods known in the art. See, e.g., Current Protocols in Molecular Biology (Ausubel et al., eds., Green Publishers Inc. and Wiley and Sons, 1994) and Harlow and Lane, *supra.* Using this approach, host cells expressing chimeric H chains (or their derivatives) are separately cultured from host cells expressing chimeric L chains (or their derivatives), and the immunoglobulin chains are separately recovered and then associated. Alternatively, the host cells can be co-cultured and the chains allowed to associate spontaneously in the culture medium, followed by recovery of the assembled immunoglobulin.

In another embodiment, a monoclonal antibody of the invention is a "humanized" antibody. Methods for humanizing non-human antibodies are well known in the art. See U.S. Pat. Nos. 5,585,089 and 5,693,762. Generally, a humanized antibody has one or more amino acid residues introduced into it from a source that is non-human. Humanization can be performed, for example, using methods described in the art (Jones et al., 1986, Nature 321:522-25; Riechmann et al., 1998, Nature 332:323- 27; Verhoeyen et al., 1988, Science 239:1534-36), by substituting at least a portion of a rodent complementarity-determining region (CDR) for the corresponding regions of a human antibody.

Techniques for creating recombinant DNA versions of the antigen-binding regions of antibody molecules (i.e., Fab or variable region fragments) which bypass the generation of monoclonal antibodies are encompassed within the scope of the present invention. In this technique, antibody-specific messenger RNA molecules are extracted from immune system cells taken from an immunized animal and transcribed into cDNA. The cDNA is then cloned into a bacterial expression system. One example of such a technique suitable for the practice of this invention uses a bacteriophage lambda vector system having a leader sequence that causes the expressed Fab protein to migrate to the periplasmic space (between the bacterial cell membrane and the cell wall) or to be secreted. One can rapidly generate and screen great numbers of functional Fab fragments for those which bind the antigen. Such (E10C)hPYY₃₋₃₆- or (D11C)hPYY₃₋₃₆-binding molecules (Fab fragments with specificity for (E10C)hPYY₃₋₃₆ or (D11C)hPYY₃₋₃₆ polypeptides) are specifically encompassed within the term "antibody" as used herein.

Also within the scope of the invention are techniques developed for the production of chimeric antibodies by splicing the genes from a mouse antibody molecule of appropriate antigen-specificity together with genes from a human antibody molecule of appropriate biological activity (such as the ability to activate human complement and mediate antibody-dependent cellular cytotoxicity (ADCC)). Morrison et al., Proc. Natl. Acad. Sci. U.S.A. 81:6851-55, 1984; Neuberger et al., Nature, 312:604-08, 1984. Selective binding agents such as antibodies produced by this technique are within the scope of the invention.

It will be appreciated that the invention is not limited to mouse or rat monoclonal antibodies; in fact, human antibodies may be used. Such antibodies can be obtained by using human hybridomas. Fully human antibodies that bind (E10C)hPYY₃₋₃₆ or (D11C)hPYY₃₋₃₆ polypeptides are thus encompassed by the invention. Such antibodies are produced by immunizing with an (E10C)hPYY₃₋₃₆ or (D11C)hPYY₃₋₃₆ antigen (optionally conjugated to a carrier) transgenic animals (e.g., mice) capable of producing a repertoire of human antibodies in the absence of endogenous immunoglobulin production. See, e.g., Jakobovits et al., Proc. Natl. Acad. Sci. U.S.A. 90:2551-55, 1993; Jakobovits et al., Nature 362:255-58, 1993; Bruggemann et al., Year in Immuno. 7:33-40, 1993.

Also encompassed by the invention are human antibodies that bind (E10C)hPYY₃₋₃₆ or (D11C)hPYY₃₋₃₆ polypeptides. Using transgenic animals (e.g., mice) that are capable of producing a repertoire of human antibodies in the absence of endogenous immunoglobulin production such antibodies are produced by immunization with an (E10C)hPYY₃₋₃₆ or (D11C)hPYY₃₋₃₆ polypeptide antigen (i.e., having at least 6 contiguous amino acids), optionally conjugated to a carrier. See, e.g., Jakobovits et al., 1993 *supra*; Jakobovits et al., Nature 362:255-58, 1993; Bruggermann et al., 1993, *supra.* In one method, such transgenic animals are produced by incapacitating the endogenous loci encoding the heavy and light immunoglobulin chains therein, and inserting loci encoding human heavy and light chain proteins into the genome thereof. Partially modified animals, that is, those having less than the full complement of modifications, are then cross-bred to obtain an animal having all of the desired immune system modifications. When administered an immunogen, these transgenic animals produce antibodies with human (rather than, e.g., murine) amino acid sequences, including variable regions which are immunospecific for these antigens. See PCT Pat. Appl. Publ. Nos.: WO 96/33735 and WO 94/02602. Additional methods are described in U.S. Pat. No. 5,545,807, PCT Pat. Appl. Publ. Nos.: WO 91/10741 and WO 90/04036, and in EP Patent No. 0 546 073 B1 and PCT Pat. Appl. Publ. No. WO 92/03918. Human antibodies can also be produced by the expression of recombinant DNA in host cells or by expression in hybridoma cells as described herein.

In an alternative embodiment, human antibodies can also be produced from phage-display libraries (Hoogenboom et al., J. Mol. Biol. 227:381, 1991; Marks et al., J. Mol. Biol. 222:581, 1991). These processes mimic immune selection through the display of antibody repertoires on the surface of filamentous bacteriophage, and subsequent selection of phage by their binding to an antigen of choice. One such technique is described in PCT Pat. Appl. Publ. No. WO 99/10494, which describes the isolation of high affinity and functional agonistic antibodies for MPL- and msk-receptors using such an approach.

Chimeric, CDR grafted, and humanized antibodies are typically produced by recombinant methods. Nucleic acids encoding the antibodies are introduced into host cells and expressed using materials and procedures described herein and known in the art. In a preferred embodiment, the antibodies are produced in mammalian host cells, such as CHO cells. Monoclonal (e.g., human) antibodies may be produced by the expression of recombinant DNA in host cells or by expression in hybridoma cells as described herein.

The anti-(E10C)hPYY₃₋₃₆ and anti-(D11C)hPYY₃₋₃₆ antibodies of the invention may be employed in any known assay method, such as competitive binding assays, direct and indirect sandwich assays, and immunoprecipitation assays (Sola, Monoclonal Antibodies: A Manual of Techniques, 147-158 (CRC Press, Inc., 1987)) for the detection and quantitation of (E10C)hPYY₃₋₃₆ and (D11C)hPYY₃₋₃₆ polypeptides, as well as polypeptide purification. The antibodies will bind (E10C)hPYY₃₋₃₆ or (D11C)hPYY₃₋₃₆ polypeptides with an affinity that is appropriate for the assay method being employed.

The PYY agonists of the invention may be provided in the form of pharmaceutically acceptable acid addition salts for use in the method aspect of the invention. Representative pharmaceutically acceptable acid addition salts of the present compounds include hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, pamoate, palmitate, malonate, stearate, laurate, malate, borate, hexafluorophosphate, naphthylate, glucoheptonate, lactobionate and laurylsulfonate salts and the like. Salts of the non-pegylated variants need not be pharmaceutically acceptable where the variant is to be used as an intermediate in the preparation of the PEG-PYY₃₋₃₆ variant conjugate.

The PYY agonists of the present invention will generally be administered in the form of a pharmaceutical composition. The pharmaceutical composition may, for example, be in a form suitable for oral administration (e.g., a tablet, capsule, pill, powder, solution, suspension), for parenteral injection (e.g.., a sterile solution, suspension or emulsion), for intranasal administration (e.g., an aerosol drops, etc), for rectal administration (e.g., a suppository) or for transdermal (e.g., a patch). The pharmaceutical composition may be in unit dosage forms suitable for single administration of precise dosages. The pharmaceutical composition will include a conventional pharmaceutical carrier and a PYY agonist of the invention as an active ingredient. In addition, it may include other pharmaceutical agents, adjuvants, etc.

Methods of preparing various pharmaceutical compositions of bioactive peptides are well known in the pharmaceutical sciences art. For example, see U.S. Pat. Appl. Publ. No. 2005/0009748 (for oral administration); and 2004/0157777, 2005/0002927 and 2005/0215475 (for transmucosal administration, e.g., intranasal or buccal administration). See also Remington: The Practice of Pharmacy, Lippincott Williams and Wilkins, Baltimore, MD, 20th ed. 2000.

The PYY agonists of this invention may be used in conjunction with other pharmaceutical agents for the treatment of the disease states or conditions described herein. Therefore methods of treatment that include administering compounds of the present invention in combination with other pharmaceutical agents are also provided by the present invention.

Suitable pharmaceutical agents that may be used in combination with the PYY agonists of the present invention include other anti-obesity agents such as cannabinoid-1 (CB-1) antagonists (such as rimonabant), 11β-hydroxy steroid dehydrogenase-1 (11β-HSD type 1) inhibitors, MCR-4 agonists, cholecystokinin-A (CCK-A) agonists, monoamine reuptake inhibitors (such as sibutramine), sympathomimetic agents, β₃ adrenergic receptor agonists, dopamine receptor agonists (such as bromocriptine), melanocyte-stimulating hormone receptor analogs, 5HT2c receptor agonists, melanin concentrating hormone antagonists, leptin, leptin analogs, leptin receptor agonists, galanin antagonists, lipase inhibitors (such as tetrahydrolipstatin, i.e. orlistat), anorectic agents (such as a bombesin agonist), neuropeptide-Y receptor antagonists (e.g., NPY Y5 receptor antagonists), thyromimetic agents, dehydroepiandrosterone or an analog thereof, glucocorticoid receptor agonists or antagonists, orexin receptor antagonists, glucagon-like peptide-1 receptor agonists, ciliary neurotrophic factors (such as Axokine^{™} available from Regeneron Pharmaceuticals, Inc., Tarrytown, NY and Procter & Gamble Company, Cincinnati, OH), human agouti-related protein (AGRP) inhibitors, ghrelin receptor antagonists, histamine 3 receptor antagonists or inverse agonists, neuromedin U receptor agonists, MTP/ApoB inhibitors (e.g., gut-selective MTP inhibitors, such as dirlotapide) and the like.

Preferred anti-obesity agents for use in combination with the PYY agonists of the present invention include CB-1 receptor antagonists, gut-selective MTP inhibitors, CCKa agonists, 5HT2c receptor agonists, NPY Y5 receptor antagonists, orlistat, and sibutramine. Preferred CB-1 receptor antagonists for use in the methods of the present invention include: rimonabant (SR141716A also known under the tradename Acomplia^{™}) is available from Sanofi-Synthelabo or can be prepared as described in U.S. Pat. No. 5,624,941; *N*-(piperidin-1-yl)-1-(2,4-dichlorophenyl)-5-(4-iodophenyl)-4-methyl-1H-pyrazole-3-carboxamide (AM251) is available from Tocris^{™}, Ellisville, MO; [5-(4-bromophenyl)-1-(2,4-dichloro-phenyl)-4-ethyl-*N*-(1-piperidinyl)-1*H*-pyrazole-3-carboxamide] (SR147778) which can be prepared as described in U.S. Pat. No. 6,645,985; *N*-(piperidin-1-yl)-4,5-diphenyl-1-methylimidazole-2-carboxamide, *N*-(piperidin-1-yl)-4-(2,4-dichlorophenyl)-5-(4-chlorophenyl)-1-methylimidazole-2-carboxamide, *N*-(piperidin-1-yl)-4,5-di-(4-methylphenyl)-1-methylimidazole-2-carboxamide, *N*-cyclohexyl-4,5-di-(4-methylphenyl)-1-methylimidazole-2-carboxamide, *N-*(cyclohexyl)-4-(2,4-dichlorophenyl)-5-(4-chlorophenyl)-1-methylimidazole-2-carboxamide, and *N*-(phenyl)-4-(2,4-dichlorophenyl)-5-(4-chlorophenyl)-1-methylimidazole-2-carboxamide which can be prepared as described in PCT Pat. Appl. Publ. No. WO 03/075660; the hydrochloride, mesylate and besylate salt of 1-[9-(4-chloro-phenyl)-8-(2-chloro-phenyl)-9H-purin-6-yl]-4-ethylamino-piperidine-4-carboxylic acid amide which can be prepared as described in U.S. Pat. Appl. Publ. No. 2004/0092520; 1-[7-(2-chloro-phenyl)-8-(4-chlorophenyl)-2-methyl-pyrazolo[1,5-a][1,3,5]triazin-4-yl]-3-ethylamino-azetidine-3-carboxylic acid amide and 1-[7-(2-chloro-phenyl)-8-(4-chloro-phenyl)-2-methyl-pyrazolo[1,5-a][1,3,5]triazin-4-yl]-3-methylamino-azetidine-3-carboxylic acid amide which can be prepared as described in U.S. Pat. Appl. Publ. No. 2004/0157839; 3-(4-chloro-phenyl)-2-(2-chloro-phenyl)-6-(2,2-difluoro-propyl)-2,4,5,6-tetrahydro-pyrazolo[3,4-c]pyridin-7-one which can be prepared as described in U.S. Pat. Appl. Publ, No. 2004/0214855; 3-(4-chloro-phenyl)-2-(2-chloro-phenyl)-7-(2,2-difluoro-propyl)-6,7-dihydro-2H,5H-4-oxa-1,2,7-triaza-azulen-8-one which can be prepared as described in U.S. Pat. Appl. Publ. No. 2005/0101592; 2-(2-chloro-phenyl)-6-(2,2,2-trifluoroethyl)-3-(4-trifluoromethyl-phenyl)-2,6-dihydro-pyrazolo[4,3-d]pyrimidin-7-one which can be prepared as described in U.S. Pat. Appl. Publ. No. 2004/0214838; (S)-4-chloro-N-{[3-(4-chloro-phenyl)-4-phenyl-4,5-dihydro-pyrazol-1-yl]-methylamino-methylene}-benzenesulfonamide (SLV-319) and (S)-N-{[3-(4-chlorophenyl)-4-phenyl-4,5-dihydro-pyrazol-1-yl]-methylamino-methylene}-4-trifluoromethyl-benzenesulfonamide (SLV-326) which can be prepared as described in PCT Pat. Appl. Publ. No. WO 02/076949; N-piperidino-5-(4-bromophenyl)-1-(2,4-dichlorophenyl)-4-ethylpyrazole-3-carboxamide which can be prepared as described in U.S. Patent No. 6,432,984; 1-[bis-(4-chloro-phenyl)-methyl]-3-[(3,5-difluoro-phenyl)-methanesulfonylmethylene]-azetidine which can be prepared as described in U.S. Pat. No. 6,518,264; 2-(5-(trifluoromethyl)pyridin-2-yloxy)-N-(4-(4-chlorophenyl)-3-(3-cyanophenyl)butan-2-yl)-2-methylpropanamide which can be prepared as described in PCT Pat. Appl. Publ. No. WO 04/048317; 4-{[6-methoxy-2-(4-methoxyphenyl)-1-benzofuran-3-yl]carbonyl}benzonitrile (LY-320135) which can be prepared as described in U.S. Pat. No. 5,747,524; 1-[2-(2,4-dichlorophenyl)-2-(4-fluorophenyl)-benzo[1,3]dioxole-5-sulfonyl]-piperidine which can be prepared as described in WO 04/013120; and [3-amino-5-(4-chlorophenyl)-6-(2,4-dichlorophenyl)-furo[2,3-b]pyridin-2-yl]-phenyl-methanone which can be prepared as described in PCT Pat. Appl. Publ. No. WO 04/012671.

Preferred intestinal-acting MTP inhibitors for use in the combinations, pharmaceutical compositions, and methods of the invention include dirlotapide ((*S*)-*N*-{2-[benzyl(methyl)amino]-2-oxo-1-phenylethyl}-1-methyl-5-[4'-(trifluoromethyl)[1,1'-biphenyl]-2-carboxamido]-1*H*-indole-2-carboxamide) and 1-methyl-5-[(4'-trifluoromethyl-biphenyl-2-carbonyl)-amino]-1H-indole-2-carboxylic acid (carbamoyl-phenyl-methyl)-amide which can both be prepared using methods described in U.S. Pat. No. 6,720,351; (S)-2-[(4'-trifluoromethyl-biphenyl-2-carbonyl)-amino]-quinoline-6-carboxylic acid (pentylcarbamoyl-phenyl-methyl)-amide, (S)-2-[(4'-tert-butyl-biphenyl-2-carbonyl)-amino]-quinoline-6-carboxylic acid {[(4-fluoro-benzyl)-methyl-carbamoyl]-phenyl-methyl}-amide, and (S)-2-[(4'-tert-butyl-biphenyl-2-carbonyl)-amino]-quinoline-6-carboxylic acid [(4-fluoro-benzylcarbamoyl)-phenyl-methyl]-amide which can all be prepared as described in U.S. Pat. Appl. Publ. No. 2005/0234099A1, (-)-4-[4-[4-[4-[[(2*S*,4*R*)-2-(4-chlorophenyl)-2-[[(4-methyl-4H-1,2,4-triazol-3-yl)sulfanyl]methyl-1,3-dioxolan-4-yl]methoxy]phenyl]piperazin-1-yl]phenyl]-2-(1*R*)-1-methylpropyl]-2,4-dihydro-3*H*-1,2,4-triazol-3-one (also known as Mitratapide or R103757) which can be prepared as described in U.S. Pat. Nos. 5,521,186 and 5,929,075; and implitapide (BAY 13-9952) which can be prepared as described in U.S. Pat. No. 6,265,431. Most preferred is dirlotapide, mitratapide, (S)-2-[(4'-trifluoromethyl-biphenyl-2-carbonyl)-amino]-quinoline-6-carboxylic acid (pentylcarbamoyl-phenyl-methyl)-amide, (S)-2-[(4'-tert-butyl-biphenyl-2-carbonyl)-amino]-quinoline-6-carboxylic acid {[(4-fluoro-benzyl)-methyl-carbamoyl]-phenyl-methyl}-amide, or (S)-2-[(4'-tert-butyl-biphenyl-2-carbonyl)-amino]-quinoline-6-carboxylic acid [(4-fluoro-benzylcarbamoyl)-phenyl-methyl]-amide. Preferred NPY Y5 receptor antagonist include: 2-oxo-N-(5-phenylpyrazinyl)spiro[isobenzofuran-1 (3H),4'-piperidine]-1'-carboxamide which can be prepared as described in U.S. Pat. Appl. Publ. No. 2002/0151456; and 3-oxo-N-(5-phenyl-2-pyrazinyl)-spiro[isobenzofuran-1(3H), 4'-piperidine]-1'-carboxamide; 3-oxo-N-(7-trifluoromethylpyrido[3,2-b]pyridin-2-yl)-spiro-[isobenzofuran-1(3H), 4'-piperidine]-1'-carboxamide; N-[5-(3-fluorophenyl)-2-pyrimidinyl]-3-oxospiro-[isobenzofuran-1(3H), [4'-piperidine]-1'-carboxamide; *trans*-3'-oxo-N-(5-phenyl-2-pyrimidinyl)] spiro[cyclohexane-1,1'(3'H)-isobenzofuran]-4-carboxamide; *trans*-3'-oxo-N-[1-(3-quinolyl)-4-imidazolyl]spiro[cyclohexane-1,1'(3'H)-isobenzofuran]-4-carboxamide; *trans*-3-oxo-N-(5-phenyl-2-pyrazinyl)spiro[4-azaiso-benzofuran-1 (3H),1'-cyclohexane]-4'-carboxamide; *trans*-N-[5-(3-fluorophenyl)-2-pyrimidinyl]-3-oxospiro[5-azaisobenzofuran-1(3H), 1'-cyclohexane]-4'-carboxamide; *trans*-N-[5-(2-fluorophenyl)-2-pyrimidinyl]-3-oxospiro[5-azaisobenzofuran-1(3H), 1'-cyclohexane]-4'-carboxamide; *trans*-N-[1-(3,5-difluorophenyl)-4-imidazolyl]-3-oxospiro[7-azaisobenzofuran-1(3H),1'-cyclohexane]-4'-carboxamide; *trans*-3-oxo-N-(1-phenyl-4-pyrazolyl)spiro[4-azaisobenzofuran-1 (3H),1'-cyclohexane]-4'-carboxamide; *trans*-N-[1-(2-fluorophenyl)-3-pyrazolyl]-3-oxospiro[6-azaisobenzofuran-1(3H),1'-cyclohexane]-4'-carboxamide; *trans*-3-oxo-N-(1-phenyl-3-pyrazolyl)spiro[6-azaisobenzofuran-1(3H),1'-cyclohexane]-4'-carboxamide; and *trans*-3-oxo-N-(2-phenyl-1,2,3-triazol-4-yl)spiro[6-azaisobenzofuran-1(3H),1'-cyclohexane]-4'-carboxamide, all of which can be prepared as described in described in PCT Pat. Appl. Publ. No. WO 03/082190; and pharmaceutically acceptable salts and esters thereof. All of the above recited U.S. patents and publications are incorporated herein by reference.

In the methods aspect of the invention, a PYY agonist of the invention, alone or in combination with one or more other pharmaceutical agents, is peripherally administered to a subject separately or together in any of the conventional methods of peripheral administration known in the art. Accordingly, the PYY agonist or combination may be administered to a subject parenterally (e.g., intravenously, intraperitoneally, intramuscularly or subcutaneously), intranasally, orally, sublingually, buccally, by inhalation (e.g., by aerosol), rectally (e.g., by suppositories) or transdermally. Parenteral administration is a preferred method of administration, and subcutaneous administration is a preferred method of parenteral administration.

Compositions suitable for parenteral injection generally include pharmaceutically acceptable sterile aqueous or nonaqueous solutions, dispersions, suspensions, or emulsions, and sterile powders for reconstitution into sterile injectable solutions or dispersions. Examples of suitable aqueous and nonaqueous carriers or diluents (including solvents and vehicles) include water, ethanol, polyols (propylene glycol, polyethylene glycol, glycerol, and the like), suitable mixtures thereof, triglycerides including vegetable oils such as olive oil, and injectable organic esters such as ethyl oleate.

These compositions for parenteral injection may also contain excipients such as preserving, wetting, solubilizing, emulsifying, and dispersing agents. Prevention of microorganism contamination of the compositions can be accomplished with various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents capable of delaying absorption, for example, aluminum monostearate and gelatin.

The PYY agonists of the present invention will be administered to a subject at a dosage that varies depending on a number of factors, including the mode of administration, the age and weight of the subject, the severity of the disease, condition or disorder being treated, and the pharmacological activity of the PYY agonist being administered. The determination of dosage ranges and optimal dosages for a particular patient is well within the ordinary skill in the art.

For parenteral administration, the PYY agonists of the present invention may be administered to a human subject at dosage levels in the range of about 0.01 µg/kg to about 10 mg/kg/dose in a dosing regimen on a non-pegylated variant basis. For example, for the 30K mPEG maleimide(E10C)hPYY₃₋₃₆, the parenteral dosing level would be in the range of about 0.01 µg/kg to about 10 mg/kg/dose in a dosing regimen on an (E10C)hPYY₃₋₃₆ basis, preferably in the range of about 0.05 mg/kg to about 1.0 mg/kg/dose, or about 0.05 or 0.1 mg/kg to about 1.0 mg/kg/dose, or about 0.05 or 0.1 mg/kg to about 0.3 or 0.5 mg/kg/dose. For example, a dose of 85 mg of 30K mPEG maleimide(E10C)hPYY₃₋₃₆, which has a molecular weight of about 34024 Da (30 k Da PEG plus 4024, the molecular weight of the non-pegylated peptide), is equivalent to 10 mg on a non-pegylated, (E10C)hPYY₃₋₃₆ basis. The dosing regimen may be one or more doses daily, preferably before a meal, or, particularly with the 30K mPEG maleimide(E10C)hPYY₃₋₃₆ or the 20K mPEG maleimide(E10C)hPYY₃₋₃₆, a dosing regimen of 2 or 3 times a week or once weekly or once every 10-14 days is preferred.

Embodiments of the present invention are illustrated by the following Examples. It is to be understood, however, that the embodiments of the invention are not limited to the specific details of these Examples, as other variations thereof will be known, or apparent in light of the instant disclosure and appendant claims, to one of ordinary skill in the art. All references cited herein are hereby incorporated by reference.

### EXAMPLES

### Example 1

### Linear 30K and 20K mPEG and 20 K Maleimide (E10C)hPYY₃₋₃₆

This example provides the preparation of substantially homogeneous monopegylated (E10C)hPYY₃₋₃₆ with mPEG (30K or 20K) attached at residue 10.

### (a) Preparation of (E10C)hPYY₃₋₃₆

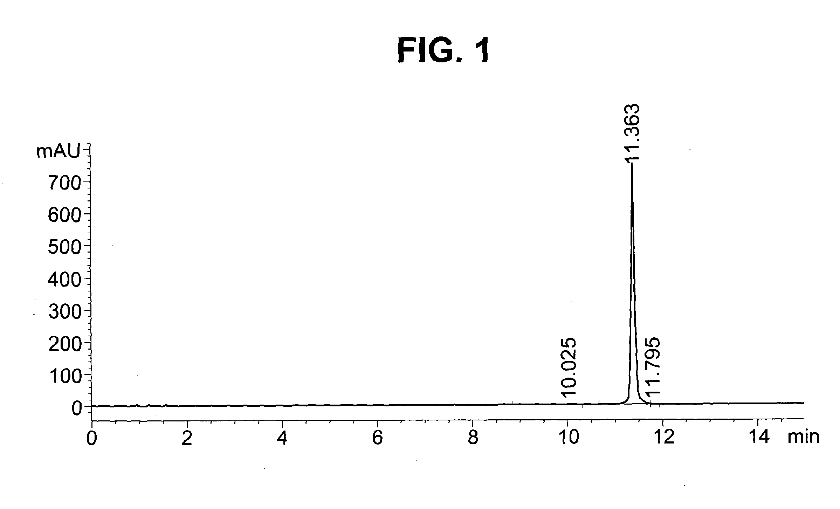

(E10C)PYY₃₋₃₆ was synthesized by solid-phase method using Fmoc strategy with 2-(1H-benzotrizole-1-yl)-1,1,3,3-tetramethyl uronium hexafluorophosphate (HBTU) activation (Fastmoc, 0.15 mmol cycles) using an automatic peptide synthesizer (model 433A; Applied Biosystems, Foster City, CA). The side chain protection groups used were Trt for Asn, Gln, Cys and His; tBu for Ser, Thr, and Tyr; Boc for Lys; OtBu for Asp and Glu; and Pbf for Arg. Cleavage of peptide-resin was completed with a mixture of 9mL of trifluoroacetic acid (TFA), 0.5 g of phenol, 0.5mL of H₂O, 0.5mL of thioanisole and 0.25mL of 1,2 ethanedithiol at room temperature for 4 h. Peptide was precipitated in ice-cold ethyl ether, and washed with ethyl ether, dissolved in DMSO and purified by reverse phase HPLC on a Waters Deltapak C18, 15um, 100Å, 50x300mmID column (Cat # WAT011801, Waters, Milford, MA) using a linear gradient from 100% Solvent A: 0% Solvent B to 70% solvent A : 30% solvent B in 30 minutes at a flow rate of 80 mL /min. Solvent A is an aqueous 0.1% TFA (trifluoroacetic acid) solution. Solvent B is 0.1% TFA solution in acetonitrile. The molecular mass of the purified peptide was confirmed by ESI-MS (M_{Avg}=4024), and purity was assessed by reversed phase HPLC (Figure 1).

### (b) Preparation of linear 30K mPEG maleimide (E10C)hPY₃₋₃₆

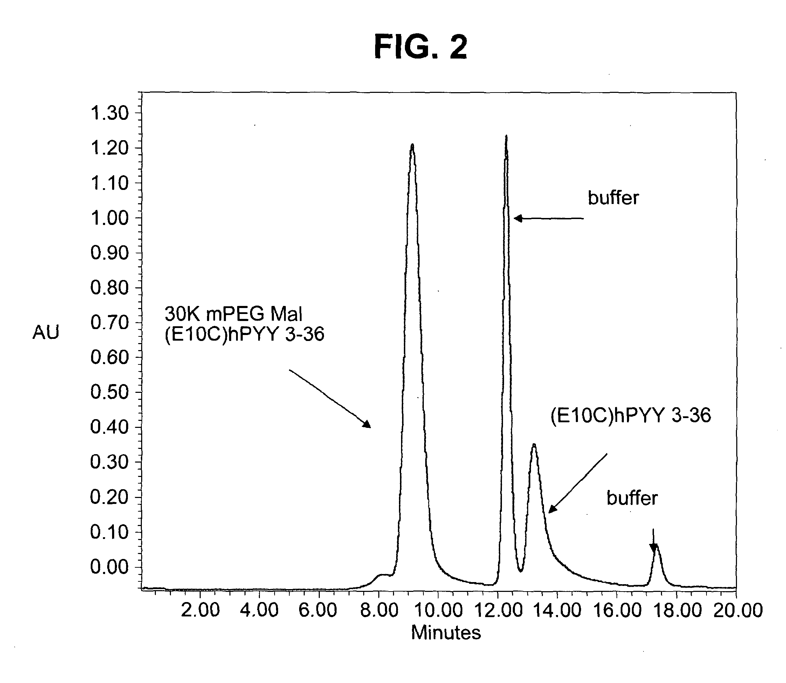

Linear mPEG maleimide reagent of approximately 30,000 MW (Sunbright ME-300MA, NOF Corporation, Tokyo, Japan) was selectively coupled to (E10C)hPYY ₃₋₃₆ on the sulfhydryl group of the cysteine at residue 10. Linear 30K mPEG maleimide, dissolved in 20 mM HEPES (Sigma Chemical, St. Louis, MO) pH 7.0, or, alternatively, 20mM sodium acetate (Sigma Chemical, St. Louis, MO) pH 4.5, was immediately reacted with (E10C)hPYY₃₋₃₆ peptide by direct addition of peptide to yield a 1 mg/mL peptide concentration and a relative mPEG:(E10C)hPYY₃₋₃₆ molar ratio of about 1:1. Reactions were carried out in the dark at room temperature for 0.5-24 hours. Reactions in HEPES pH 7.0 were stopped by dilution into 20mM sodium acetate pH 4.5, for immediate purification on cation exchange chromatography. Reactions in 20mM sodium acetate, pH 4.5, were loaded directly onto cation exchange chromatography. Reaction products were assessed by SEC-HPLC (Figure 2).

### (c) Purification of linear 30K mPEG maleimide (E10C)hPYY₃₋₃₆

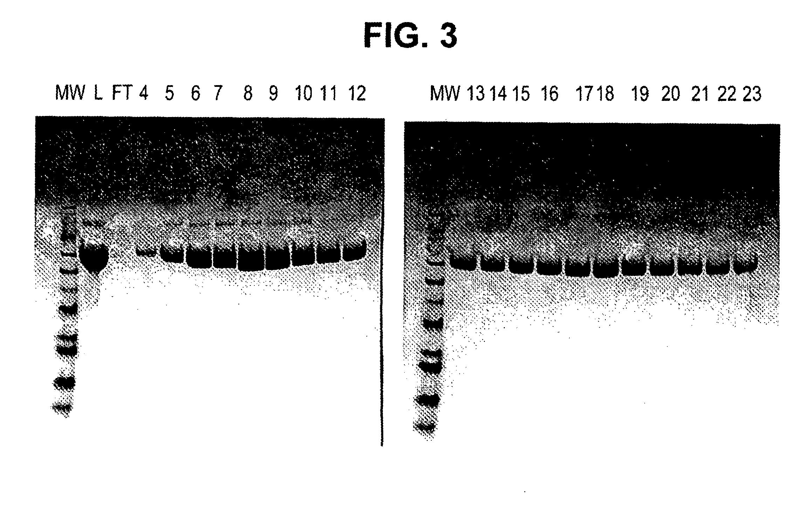

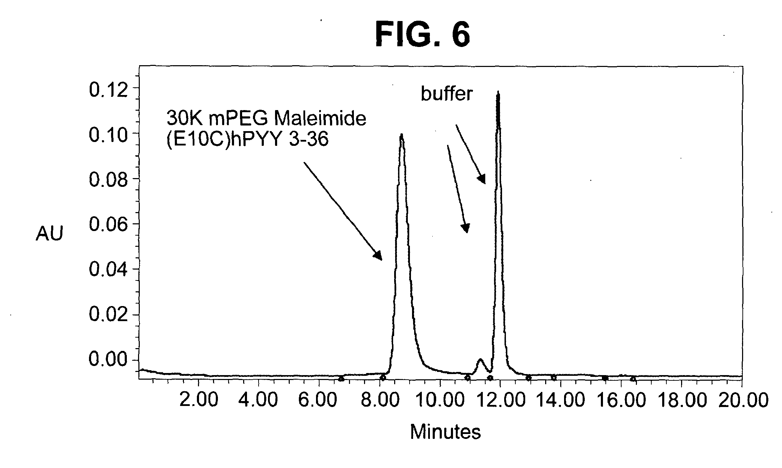

The pegylated (E10C)hPYY₃₋₃₆ species was purified from the reaction mixture to >95% using a single ion exchange chromatography step. Mono-pegylated (E10C)hPYY₃₋₃₆ was purified from unmodified (E10C)hPYY₃₋₃₆ and larger molecular weight species using cation exchange chromatography. A typical linear 30K mPEG maleimide (E10C)hPYY₃₋₃₆ reaction mixture (10 mg protein), as described above, was fractionated on a SP-Sepharose Hitrap column (5 mL)(Amersham Pharmacia Biotech, GE Healthcare, Piscataway, NJ) equilibrated in 20 mM sodium acetate, pH 4.5 (Buffer A). The reaction mixture was diluted 7X with buffer A and loaded onto the column at a flow rate of 2.5 mL/min. The column was washed with 5-10 column volumes of buffer A. Subsequently, the various (E10C)hPYY₃₋₃₆ species were eluted from the column in 20 column volumes of a linear NaCI gradient of 0-100 mM. The eluant was monitored by absorbance at 280 nm (A₂₈₀) and appropriate size fractions were collected. Fractions were pooled as to extent of pegylation, as assessed by ^{®}SDS-PAGE (Figure 3). The purified pool was then concentrated to 0.5-5 mg/mL in a Centriprep 3 concentrator (Amicon Technology Corporation, Northborough, MA) or, alternatively, using a Vivaspin 10K concentrator (Vivascience Sartorius Group, Hannover, Germany). Protein concentration of the purified pool was determined by comparing the RP HPLC peak area to a PYY₃₋₃₆ standard curve (not shown) or, alternatively, the concentration of the purified pool was determined by the absorbance at 280nm using an experimentally derived extinction coefficient. A purified pool of pegylated (E10C)hPYY₃₋₃₆ was profiled using SEC-HPLC as shown in Figure 6.

### (d) Preparation of linear 20K mPEG maleimide (E10C)hPYY₃₋₃₆

Linear mPEG maleimide reagent of approximately 20,000 MW (Sunbright ME-200MA, NOF Corporation) was selectively coupled to (E10C)hPYY ₃₋₃₆ on the sulfhydryl group of the cysteine at residue 10. Linear 20K mPEG maleimide, dissolved 20mM sodium acetate (Sigma Chemical, St. Louis, MO) pH 4.5, was immediately reacted with (E10C)hPYY₃₋₃₆ peptide by direct addition of peptide to yield a 1 mg/mL peptide concentration and a relative mPEG:(E10C)hPYY₃₋₃₆ molar ratio of about 1.3:1. Reactions were carried out in the dark at room temperature for 60 minutes followed by 16 hours at 4°C. Reactions in 20 mM sodium acetate, pH 4.5, were loaded directly onto cation exchange chromatography. Reaction products were assessed by SEC-HPLC.

### (e) Purification of linear 20K mPEG maleimide (E10C)hPYY₃₋₃₆

The pegylated (E10C)hPYY₃₋₃₆ species was purified from the reaction mixture to >95% using a single ion exchange chromatography step. Mono-pegylated (E10C)hPYY₃₋₃₆ was separated from free PEG, unmodified (E10C)hPYY₃₋₃₆ and larger molecular weight species using cation exchange chromatography. A typical linear 20K mPEG maleimide (E10C)hPYY₃₋₃₆ reaction mixture (20 mg protein), as described above, was fractionated on a SP-Sepharose Hitrap column (5 mL)(Amersham Pharmacia Biotech,GE Healthcare) equilibrated in 20 mM sodium acetate, pH 4.5 (Buffer A). The reaction mixture was loaded onto the column at a flow rate of 1.0 mL/min. The column was washed with 4 column volumes of buffer A at a flow rate of 2.5 mL/min. Subsequently, the various (E10C)hPYY₃₋₃₆ species were eluted from the column in 25 column volumes of a linear NaCI gradient of 0-200 mM at a flow rate of 2.5 mL/min. The eluant was monitored by absorbance at 280 nm (A₂₈₀) and appropriate size fractions were collected. Fractions were pooled as to extent of pegylation, as assessed by SEC-HPLC. The purified pool was then concentrated to 0.5-5 mg/mL using a Vivaspin 10K concentrator (Vivascience Sartorius Group). Protein concentration of the purified pool was determined by the absorbance at 280 nm using an experimentally derived extinction coefficient. The total process yield of purified mono 20K mPEG maleimide (E10C)hPYY₃₋₃₆ was 38 %. The purified pool of mono 20K mPEG maleimide (E10C)hPYY₃₋₃₆ was determined to be 96% pure using SEC-HPLC.

### Example 2

### Linear 30K mPEG Maleimide (D11C)hPYY₃₋₃₆

This example demonstrates the preparation of substantially homogeneous monopegylated (D11C)hPYY₃₋₃₆ with mPEG attached at residue 11.

### (a) Preparation of (D11C)hPYY₃₋₃₆

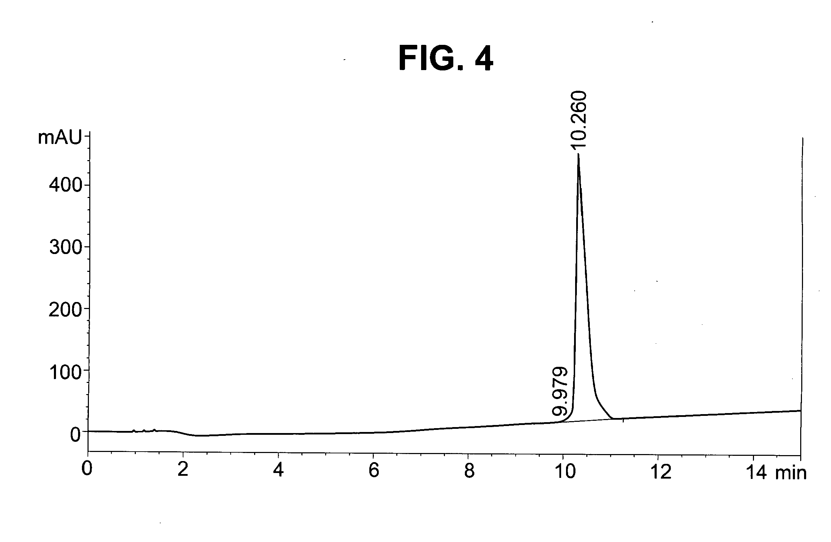

(D11C)PYY₃₋₃₆ was synthesized by solid-phase method using Fmoc strategy with 2-(1H-benzotrizole-1-yl)-1,1,3,3-tetramethyl uronium hexafluorophosphate (HBTU) activation (Fastmoc, 0.15 mmol cycles) using an automatic peptide synthesizer (model 433A; Applied Biosystems, Foster City, CA). The side chain protection groups used were Trt for Asn, Gln, Cys and His; tBu for Ser, Thr, and Tyr; Boc for Lys; OtBu for Asp and Glu; and Pbf for Arg. Cleavage of peptide-resin was completed with a mixture of 9mL of trifluoroacetic acid (TFA), 0.5 g of phenol, 0.5mL of H₂O, 0.5mL of thioanisole and 0.25mL of 1,2 ethanedithiol at room temperature for 4 h. Peptide was precipitated in ice-cold ethyl ether, and washed with ethyl ether, dissolved in DMSO and purified by reverse phase HPLC on a Waters Deltapak C18, 15um, 100Å, 50x300mmID column (Cat # WAT011801, Waters, Milford, MA) using a linear gradient from 100% Solvent A: 0% Solvent B to 70% solvent A : 30% solvent B in 30 minutes at a flow rate of 80 mL /min. Solvent A is an aqueous 0.1% TFA (trifluoroacetic acid) solution. Solvent B is 0.1% TFA solution in acetonitrile. The molecular mass of the purified peptide was confirmed by ESI-MS (M_{Avg}=4038), and purity was assessed by reversed phase HPLC (Figure 4).

### (b) Preparation of linear 30K mPEG maleimide (D11C)hPYY₃₋₃₆

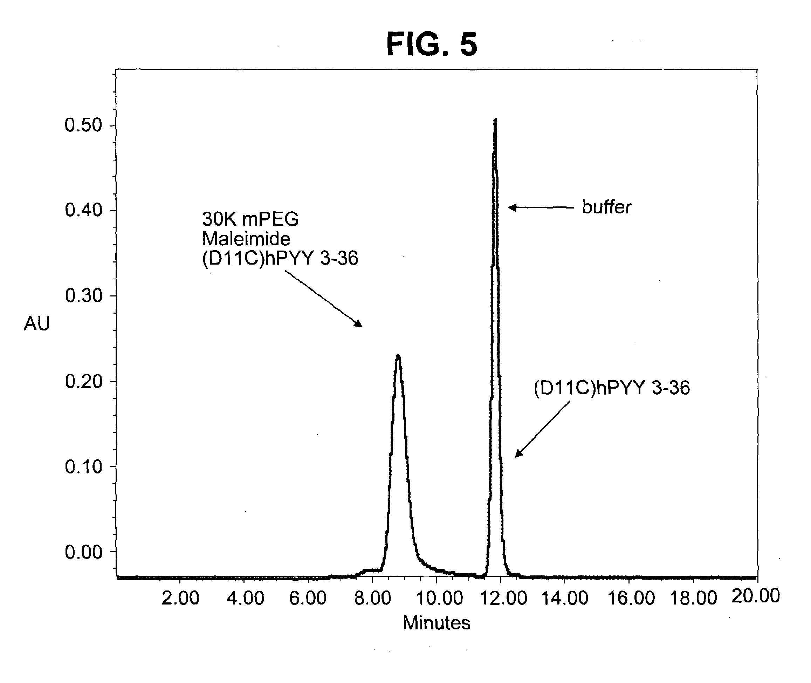

Linear mPEG maleimide reagent of approximately 30,000 MW (Sunbright ME-300MA, NOF Corporation, Tokyo, Japan) was selectively coupled to (D11C)hPYY₃₋₃₆ on the sulfhydryl group of the cysteine at residue 11. Linear 30K mPEG maleimide, dissolved in 20 mM HEPES (Sigma Chemical, St. Louis, MO) pH 7.0 was immediately reacted with (D11C)hPYY₃₋₃₆ peptide by direct addition of peptide to yield a 1 mg/mL peptide concentration and a relative mPEG:(D11C)hPYY₃₋₃₆ molar ratio of about 1:1. Reactions were carried out in the dark at room temperature for 0.5-24 hours. Reactions were stopped by dilution into 20mM sodium acetate pH 4.5, for immediate purification on cation exchange chromatography. Reaction products were assessed by SEC-HPLC (Figure 5).

Alternatively, instead of dissolving the linear 30K mPEG maleimide in HEPES as described above, it is dissolved in 20 mM sodium acetate (Sigma Chemical, St. Louis, MO), pH 4.5, and is immediately reacted with (D11C)hPYY₃₋₃₆ peptide by direct addition of peptide to yield a 1 mg/mL peptide concentration and a relative mPEG:(D11C)hPYY₃₋₃₆ molar ratio of about 1:1. Reactions are carried out in the dark at room temperature for 0.5-24 hours. Reactions are loaded directly onto cation exchange chromatography. Reaction products are assessed by SEC-HPLC.

### (c) Purification of linear 30K mPEG maleimide (D11C)hPYY₃₋₃₆

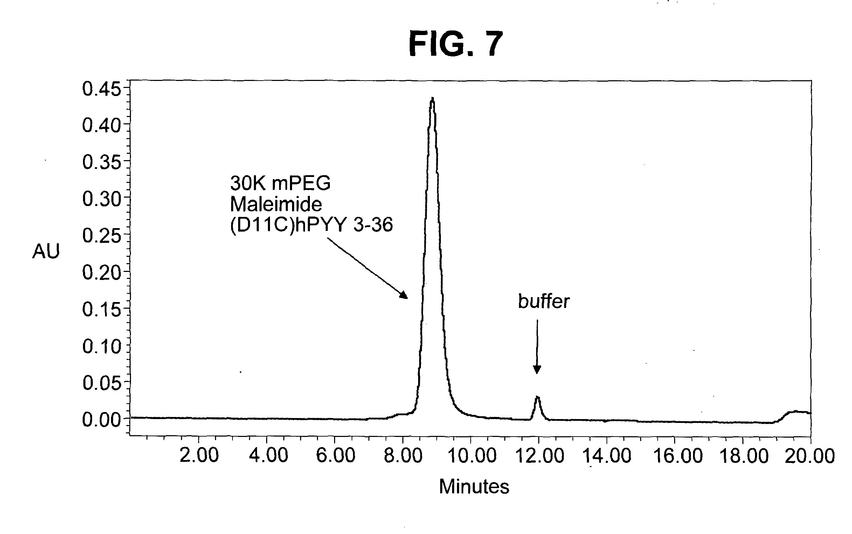

The pegylated (D11C)hPYY₃₋₃₆ species was purified from the reaction mixture to >95% using a single ion exchange chromatography step. Mono-pegylated (D11C)hPYY₃₋₃₆ was purified from unmodified (D11C)hPYY₃₋₃₆ and larger molecular weight species using cation exchange chromatography. A typical linear 30K mPEG maleimide (D11C)hPYY₃₋₃₆ reaction mixture (10 mg protein), as described above, was fractionated on a SP-Sepharose Hitrap column (5 mL)(Amersham Pharmacia Biotech,GE Healthcare, Piscataway, NJ) equilibrated in 20 mM sodium acetate, pH 4.5 (Buffer A). The reaction mixtures at pH 7.0 were diluted 7X with buffer A and loaded onto the column at a flow rate of 2.5 mL/min. The column was washed with 5-10 column volumes of buffer A. Subsequently, the various (D11C)hPYY₃₋₃₆ species were eluted from the column in 20 column volumes of a linear NaCl gradient of 0-100 mM. The eluant was monitored by absorbance at 280 nm (A₂₈₀) and appropriate size fractions were collected. Fractions were pooled as to extent of pegylation, as assessed by SEC HPLC. The purified pool was then concentrated to 0.5-5 mg/mL in a Vivaspin 10K concentrator (Vivascience Sartorius Group). Protein concentration of the purified pool was determined by comparing the RP HPLC peak area to a PYY₃₋₃₆ standard curve (not shown). A purified pool of pegylated (D11C)hPYY₃₋₃₆ was profiled using SEC-HPLC as shown in Figure 7.

Alternatively, reactions at pH 4.5, from (b) above, are loaded directly onto the column at a flow rate of 2.5 mL/min and concentration of the purified pool is determined by the absorbance at 280 nm using an experimentally derived extinction coefficient.

### Example 3

### Branched 43K mPEG Maleimide (E10C)hPYY₃₋₃₆

This example demonstrates the preparation of substantially homogeneous monopegylated (E10C)hPYY₃₋₃₆ with mPEG attached at residue 10.

### (a) Preparation of branched 43K mPEG maleimide (E10C)hPYY₃₋₃₆

Branched mPEG maleimide reagent of approximately 43,000 MW (Sunbright GL2-400MA, NOF Corporation, Tokyo, Japan) was selectively coupled to (E10C)hPYY₃₋₃₆, prepared as described in Example 1(a), on the sulfhydryl group of the cysteine at residue 10.

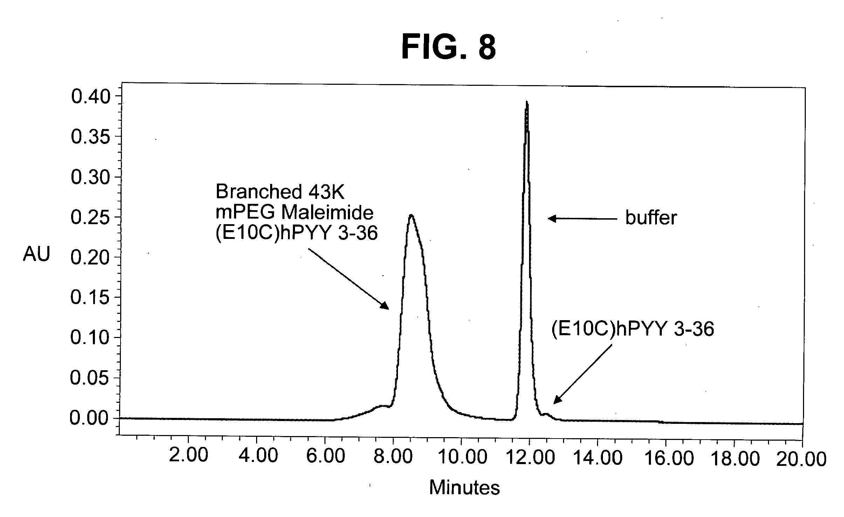

Branched 43K mPEG maleimide, dissolved in 20 mM HEPES (Sigma Chemical, St. Louis, MO), pH 7.0, was immediately reacted with (E10C)hPYY₃₋₃₆ peptide by direct addition of peptide to yield a 1 mg/mL peptide concentration and a relative mPEG:(E10C)hPYY₃₋₃₆ molar ratio of about 1:1. Reactions were carried out in the dark at room temperature for 0.5-24 hours. Reactions in HEPES, pH 7.0, were stopped by dilution into 20mM sodium acetate, pH 4.5, for immediate purification on cation exchange chromatography. Reaction products were assessed by SEC-HPLC (Figure 8).

Alternatively, branched 43K mPEG maleimide, is dissolved in 20mM sodium acetate (Sigma Chemical, St. Louis, MO), pH 4.5, and is immediately reacted with (E10C)hPYY₃₋₃₆ peptide by direct addition of peptide to yield a 1 mg/mL peptide concentration and a relative mPEG:(E10C)hPYY₃₋₃₆ molar ratio of about 1:1. Reactions are loaded directly onto cation exchange chromatography.

### (b) Purification of mono pegylated branched 43K mPEG maleimide (E10C)hPYY₃₋₃₆

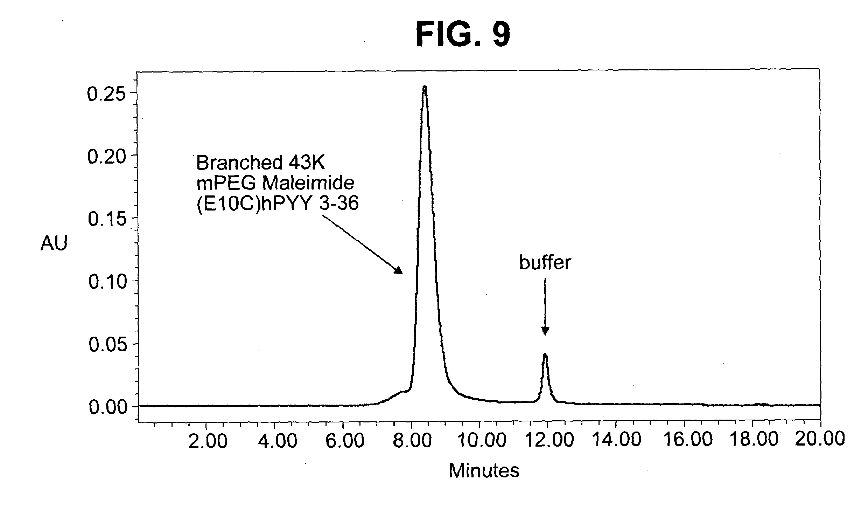

The mono pegylated branched 43K mPEG maleimide (E10C)hPYY₃₋₃₆ species was separated from unmodified (E10C)hPYY₃₋₃₆ and larger molecular weight species using a single cation exchange chromatography step. A typical branched 43K mPEGmaleimide (E10C)hPYY₃₋₃₆ reaction mixture (10 mg protein), as described above, was fractionated on a SP-Sepharose Hitrap column (5 mL)(Amersham Pharmacia Biotech, GE Healthcare) equilibrated in 20 mM sodium acetate, pH 4.5 (Buffer A). The reaction mixtures at pH 7.0 were diluted 10X with buffer A and loaded onto the column at a flow rate of 2.5 mL/min. The column was washed with 5-10 column volumes of buffer A. Subsequently, the various (E10C)hPYY₃₋₃₆ species were eluted from the column in 20 column volumes of a linear NaCl gradient of 0-100 mM. The eluant was monitored by absorbance at 280 nm (A₂₈₀) and appropriate size fractions were collected. Fractions were pooled as to extent of pegylation, as assessed by SEC-HPLC. The purified pool was then concentrated to 0.5-5 mg/mL in a Centriprep 3 concentrator (Amicon Technology Corporation) or, alternatively, using a Vivaspin 10K concentrator (Vivascience Sartorius Group). Protein concentration of the purified pool was quantitated by amino acid analysis. A purified pool of monopegylated branched 43 K mPEG maleimide (E10C)hPYY₃₋₃₆ was profiled using SEC-HPLC as shown in Figure 9.

Alternatively, protein concentration is determined by comparing the RP HPLC peak area to a PYY₃₋₃₆ standard curve (not shown) or by the absorbance at 280 nm using an experimentally derived extinction coefficient.

### Example 4

This example contemplates the preparation of substantially homogeneous monopegylated (E10C)hPYY₃₋₃₆ with linear 12 kD mPEG, or branched 20 kD mPEG, attached at residue 10, and the contemplated preparation of substantially homogeneous monopegylated (D11C)hPYY₃₋₃₆ with linear 20 kD mPEG, linear 12 kD mPEG, or branched 20 kD mPEG, attached at residue 11.

### (a) Preparation of linear 12K mPEG maleimide (E10C)hPYY₃₋₃₆

Linear mPEG maleimide reagent of approximately 12,000 MW (Sunbright ME-120MA, NOF Corporation) is selectively coupled to (E10C)hPYY₃₋₃₆ on the sulfhydryl group of the cysteine at residue 10. Linear 12K mPEG maleimide, dissolved 20mM sodium acetate (Sigma Chemical) pH 4.5, is immediately reacted with (E10C)hPYY₃₋₃₆ peptide by direct addition of peptide to yield a 1 mg/mL peptide concentration and a relative mPEG:(E10C)hPYY₃₋₃₆ molar ratio of about 1:1. Reactions are carried out in the dark at room temperature for 0.5 to 24 hours. Reactions in 20 mM sodium acetate, pH 4.5, are loaded directly onto cation exchange chromatography. Reaction products are assessed by SEC-HPLC or SDS-PAGE.

### (b) Purification of linear 12K mPEG maleimide (E10C)hPYY₃₋₃₆

The pegylated (E10C)hPYY₃₋₃₆ species is purified from the reaction mixture to >95% using a single ion exchange chromatography step. Mono-pegylated (E10C)hPYY₃₋₃₆ is separated from free PEG, unmodified (E10C)hPYY₃₋₃₆ and larger molecular weight species using cation exchange chromatography. A typical linear 12K mPEG maleimide (E10C)hPYY₃₋₃₆ reaction mixture (10 mg protein), as described above, is fractionated on a SP-Sepharose Hitrap column (5 mL)(Amersham Pharmacia Biotech,GE Healthcare) equilibrated in 20 mM sodium acetate, pH 4.5 (Buffer A). The reaction mixture is loaded onto the column at a flow rate of 1.0 mL/min. The column is washed with 4 column volumes of buffer A at a flow rate of 2.5 mL/min. Subsequently, the various (E10C)hPYY₃₋₃₆ species are eluted from the column in 25 column volumes of a linear NaCl gradient of 0-200 mM at a flow rate of 2.5 mL/min. The eluant is monitored by absorbance at 280 nm (A₂₈₀) and appropriate size fractions are collected. Fractions are pooled as to extent of pegylation, as assessed by SEC-HPLC. The purified pool is then concentrated to 0.5-5 mg/mL using a Vivaspin 10K concentrator (Vivascience Sartorius Group). Protein concentration of the purified pool is determined by the absorbance at 280 nm using an experimentally derived extinction coefficient. A purified pool of 12K mPEG maleimide (E10C)hPYY₃₋₃₆ is profiled using SEC-HPLC or SDS-PAGE.

### (c) Preparation of branched 20K mPEG maleimide (E10C)hPYY₃₋₃₆

Branched mPEG maleimide reagent of approximately 20,000 MW (Sunbright GL2-200MA, NOF Corporation) is selectively coupled to (E10C)hPYY₃₋₃₆ on the sulfhydryl group of the cysteine at residue 10. Branched 20K mPEG maleimide, dissolved in 20 mM sodium acetate (Sigma Chemical, St. Louis, MO) pH 4.5, is immediately reacted with (E10C)hPYY₃₋₃₆ peptide by direct addition of peptide to yield a 1 mg/mL peptide concentration and a relative mPEG:(E10C)hPYY₃₋₃₆ molar ratio of about 1:1. Reactions are carried out in the dark at room temperature for 0.5 to 24 hours. Reactions in 20 mM sodium acetate, pH 4.5, are loaded directly onto cation exchange chromatography. Reaction products are assessed by SEC-HPLC or SDS-PAGE.

### (d) Purification of branched 20K mPEG maleimide (E10C)hPYY₃₋₃₆

The pegylated (E10C)hPYY₃₋₃₆ species is purified from the reaction mixture to >95% using a single ion exchange chromatography step. Mono-pegylated (E10C)hPYY₃₋₃₆ is separated from free PEG, unmodified (E10C)hPYY₃₋₃₆ and larger molecular weight species using cation exchange chromatography. A typical branched 20K mPEG maleimide (E10C)hPYY₃₋₃₆ reaction mixture (10 mg protein), as described above, is fractionated on a SP-Sepharose Hitrap column (5 mL)(Amersham Pharmacia Biotech,GE Healthcare) equilibrated in 20 mM sodium acetate, pH 4.5 (Buffer A). The reaction mixture is loaded onto the column at a flow rate of 1.0 mL/min. The column is washed with 4 column volumes of buffer A at a flow rate of 2.5 mL/min. Subsequently, the various (E10C)hPYY₃₋₃₆ species are eluted from the column in 25 column volumes of a linear NaCl gradient of 0-200 mM at a flow rate of 2.5 mL/min. The eluant is monitored by absorbance at 280 nm (A₂₈₀) and appropriate size fractions are collected. Fractions are pooled as to extent of pegylation, as assessed by SEC-HPLC. The purified pool is then concentrated to 0.5-5 mg/mL using a Vivaspin 10K concentrator (Vivascience Sartorius Group). Protein concentration of the purified pool is determined by the absorbance at 280 nm using an experimentally derived extinction coefficient. A purified pool of branched 20K mPEG maleimide (E10C)hPYY₃₋₃₆ is profiled using SEC-HPLC or SDS-PAGE.

### (e) Preparation of linear 20K mPEG maleimide (D11C)hPYY₃₋₃₆

Linear mPEG maleimide reagent of approximately 20,000 MW (Sunbright ME-200MA, NOF Corporation) is selectively coupled to (D11C)hPYY₃₋₃₆ on the sulfhydryl group of the cysteine at residue 11. Linear 20K mPEG maleimide, dissolved in 20 mM sodium acetate (Sigma Chemical) pH 4.5, is immediately reacted with (D11C)hPYY₃₋₃₆ peptide by direct addition of peptide to yield a 1 mg/mL peptide concentration and a relative mPEG:(D11C)hPYY₃₋₃₆ molar ratio of about 1:1. Reactions are carried out in the dark at room temperature for 0.5 to 24 hours. Reactions in 20 mM sodium acetate, pH 4.5, are loaded directly onto cation exchange chromatography. Reaction products were assessed by SEC-HPLC or SDS-PAGE.

### (f) Purification of linear 20K mPEG maleimide (D11C)hPYY₃₋₃₆

The pegylated (D11C)hPYY₃₋₃₆ species is purified from the reaction mixture to >95% using a single ion exchange chromatography step. Mono-pegylated (D11C)hPYY₃₋₃₆ is separated from free PEG, unmodified (D11C)hPYY₃₋₃₆ and larger molecular weight species using cation exchange chromatography. A typical linear 20K mPEG maleimide (D11C)hPYY₃₋₃₆ reaction mixture (10 mg protein), as described above, is fractionated on a SP-Sepharose Hitrap column (5 mL)(Amersham Pharmacia Biotech,GE Healthcare) equilibrated in 20 mM sodium acetate, pH 4.5 (Buffer A). The reaction mixture is loaded onto the column at a flow rate of 1.0 mL/min. The column is washed with 4 column volumes of buffer A at a flow rate of 2.5 mL/min. Subsequently, the various (D11C)hPYY₃₋₃₆ species are eluted from the column in 25 column volumes of a linear NaCl gradient of 0-200 mM at a flow rate of 2.5 mL/min. The eluant is monitored by absorbance at 280 nm (A₂₈₀) and appropriate size fractions are collected. Fractions are pooled as to extent of pegylation, as assessed by SEC-HPLC. The purified pool is then concentrated to 0.5-5 mg/mL using a Vivaspin 10K concentrator (Vivascience Sartorius Group). Protein concentration of the purified pool is determined by the absorbance at 280 nm using an experimentally derived extinction coefficient. A purified pool of 20K mPEG maleimide (D11C)hPYY₃₋₃₆ is profiled using SEC-HPLC or SDS-PAGE.

### (g) Preparation of linear 12K mPEG maleimide (D11C)hPYY₃₋₃₆

Linear mPEG maleimide reagent of approximately 12,000 MW (Sunbright ME-120MA, NOF Corporation) is selectively coupled to (D11C)hPYY₃₋₃₆ on the sulfhydryl group of the cysteine at residue 10. Linear 12K mPEG maleimide, dissolved 20mM sodium acetate (Sigma Chemical, St. Louis, MO) pH 4.5, is immediately reacted with (D11C)hPYY₃₋₃₆ peptide by direct addition of peptide to yield a 1 mg/mL peptide concentration and a relative mPEG:(D11C)hPYY₃₋₃₆ molar ratio of about 1:1. Reactions are carried out in the dark at room temperature for 0.5 to 24 hours. Reactions in 20 mM sodium acetate, pH 4.5, are loaded directly onto cation exchange chromatography. Reaction products are assessed by SEC-HPLC or SDS-PAGE.

### (h) Purification of linear 12K mPEG maleimide (D11C)hPYY₃₋₃₆

The pegylated (D11C)hPYY₃₋₃₆ species is purified from the reaction mixture to >95% using a single ion exchange chromatography step. Mono-pegylated (D11C)hPYY₃₋₃₆ is separated from free PEG, unmodified (D11C)hPYY₃₋₃₆ and larger molecular weight species using cation exchange chromatography. A typical linear 12K mPEG maleimide (D11C)hPYY₃₋₃₆ reaction mixture (10 mg protein), as described above, is fractionated on a SP-Sepharose Hitrap column (5 mL)(Amersham Pharmacia Biotech,GE Healthcare) equilibrated in 20 mM sodium acetate, pH 4.5 (Buffer A). The reaction mixture is loaded onto the column at a flow rate of 1.0 mL/min. The column is washed with 4 column volumes of buffer A at a flow rate of 2.5 mL/min. Subsequently, the various (D11C)hPYY₃₋₃₆ species are eluted from the column in 25 column volumes of a linear NaCl gradient of 0-200 mM at a flow rate of 2.5 mL/min. The eluant is monitored by absorbance at 280 nm (A₂₈₀) and appropriate size fractions are collected. Fractions are pooled as to extent of pegylation, as assessed by SEC-HPLC. The purified pool is then concentrated to 0.5-5 mg/mL using a Vivaspin 10K concentrator (Vivascience Sartorius Group). Protein concentration of the purified pool is determined by the absorbance at 280 nm using an experimentally derived extinction coefficient. A purified pool of 12K mPEG maleimide (D11C)hPYY₃₋₃₆ is profiled using SEC-HPLC or SDS-PAGE.

### (i) Preparation of branched 20K mPEG maleimide (D11C)hPYY₃₋₃₆

Branched mPEG maleimide reagent of approximately 20,000 MW (Sunbright GL2-200MA, NOF Corporation) is selectively coupled to (D11C)hPYY₃₋₃₆ on the sulfhydryl group of the cysteine at residue 10. Branched 20K mPEG maleimide, dissolved in 20 mM sodium acetate (Sigma Chemical, St. Louis, MO) pH 4.5, is immediately reacted with (D11C)hPYY₃₋₃₆ peptide by direct addition of peptide to yield a 1 mg/mL peptide concentration and a relative mPEG:(D11C)hPYY₃₋₃₆ molar ratio of about 1:1. Reactions are carried out in the dark at room temperature for 0.5 to 24 hours. Reactions in 20 mM sodium acetate, pH 4.5, are loaded directly onto cation exchange chromatography. Reaction products are assessed by SEC-HPLC or SDS-PAGE.

### (j) Purification of branched 20K mPEG maleimide (D11C)hPYY₃₋₃₆

The pegylated (D11C)hPYY₃₋₃₆ species is purified from the reaction mixture to >95% using a single ion exchange chromatography step. Mono-pegylated (D11C)hPYY₃₋₃₆ is separated from free PEG, unmodified (D11C)hPYY₃₋₃₆ and larger molecular weight species using cation exchange chromatography. A typical branched 20K mPEG maleimide (D11C)hPYY₃₋₃₆ reaction mixture (10 mg protein), as described above, is fractionated on a SP-Sepharose Hitrap column (5 mL)(Amersham Pharmacia Biotech, GE Healthcare) equilibrated in 20 mM sodium acetate, pH 4.5 (Buffer A). The reaction mixture is loaded onto the column at a flow rate of 1.0 mL/min. The column is washed with 4 column volumes of buffer A at a flow rate of 2.5 mUmin. Subsequently, the various (D11C)hPYY₃₋₃₆ species are eluted from the column in 25 column volumes of a linear NaCl gradient of 0-200 mM at a flow rate of 2.5 mL/min. The eluant is monitored by absorbance at 280 nm (A₂₈₀) and appropriate size fractions are collected. Fractions are pooled as to extent of pegylation, as assessed by SEC-HPLC. The purified pool is then concentrated to 0.5-5 mg/mL using a Vivaspin 10K concentrator (Vivascience Sartorius Group). Protein concentration of the purified pool is determined by the absorbance at 280 nm using an experimentally derived extinction coefficient. A purified pool of branched 20K mPEG maleimide (D11C)hPYY₃₋₃₆ is profiled using SEC-HPLC or SDS-PAGE.

### Example 5

### Biochemical Characterization

(E10C)hPYY₃₋₃₆, (D11C)hPYY₃₋₃₆ and pegylated forms of (E10C)hPYY₃₋₃₆ and (D11C)hPYY₃₋₃₆ were characterized by various biochemical methods including Electrospray Mass Spectrometry (ESI-MS), SDS-PAGE, and SEC HPLC and RP HPLC, respectively.
(A) Electrospray ionization mass spectrometry (ESI-MS) was carried out on a 1100 series LC/MSD electrospray mass spectrometer (Agilent Technologies, Palo Alto, CA) in the positive mode. (Example 1(a), 2(a)).
(B) Reversed phase chromatography was carried out for analysis of (E10C)hPYY₃₋₃₆ peptide (Figures 1 and 4) on a ZORBAX Eclipse XDB-C8, 4.6X150mm, 5mm column (Cat # 993967-906, Agilent Technologies, Palo Alto, CA) using a linear gradient from 100 % solvent A, 0 % solvent B to 95% solvent A , 5% solvent B in 3 minutes, then from 95 % Solvent A, 5 % Solvent B to 50% solvent A, 50% solvent B in 12 minutes at a rate of 1.5 ml/minute (Example 1 (a)). Solvent A is an aqueous 0.1% TFA solution. Solvent B is 0.1% TFA solution in acetonitrile.
   Reversed phase chromatography for quantitation of pegylated (E10C)hPYY₃₋₃₆ and (D11C)PYY₃₋₃₆ (not shown) was carried out on a Vydac C18 (2.1x250 mm) column (Cat # 218MS552, Vydac, Hesperia, CA) using a linear gradient from 80% solvent A, 20% solvent B to 40% solvent A, 60% solvent B in 48 minutes at a flow rate of 0.2 mL/minute. (Example 1C, 2C) Solvent A is an aqueous, 0.1% TFA solution. Solvent B is 0.085% TFA solution in acetonitrile.
(C) Size Exclusion High Performance Liquid Chromatography (SEC-HPLC)
   The reaction mixtures of linear 30K or branched 43 K mPEG with either (E10C)hPYY₃₋₃₆ or (D11C)hPYY₃₋₃₆, their cation exchange purification pools, and final purified products were assessed using non-denaturing SEC-HPLC (Example 1(b) and (c), 2(b) and (c)). Analytical non-denaturing SEC-HPLC was carried out using a Shodex KW804 or TSK G4000PWXL (Tosohaas) in 20 mM phosphate pH 7.4, 150 mM NaCl, at a flow rate of 1.0 mL/minute (optionally Superdex 200 7.8 mm X 30 cm, Amersham Bioscience, Piscataway, NJ). Pegylation greatly increases the hydrodynamic volume of the protein resulting in a shift to an earlier retention time. In the 30K mPEG maleimide plus (E10C)hPYY₃₋₃₆ reaction mixtures, a small peak was observed corresponding to residual unmodified (E10C)hPYY₃₋₃₆, as well as new peaks corresponding to pegylated peptide species (Figure 2). New species were observed in the 30K mPEG (D11C)hPYY₃₋₃₆ and branched 43K mPEG (E10C)hPYY₃₋₃₆ reaction mixtures with very little unmodified (D11C)hPYY₃₋₃₆ or (E10C)hPYY₃₋₃₆ remaining (Figure 5 and 8). These pegylated and non-pegylated species were fractionated by SP-Sepharose chromatography, and the resultant purified mono mPEG (E10C)hPYY₃₋₃₆ and mPEG (D11C)hPYY₃₋₃₆ species were subsequently shown to elute as a single peak on non-denaturing SEC > 95% purity, (Figures 6, 7 and 9). The SP-Sepharose chromatography step effectively removed free mPEG, (E10C)hPYY₃₋₃₆ or (D11C)hPYY₃₋₃₆ and larger molecular weight species from the monopegylated linear 30K and branched 43K mPEG(E10C)hPVY₃₋₃₆ or mPEG (D11C)hPYY₃₋₃₆.
(D) SDS PAGE
   SDS-PAGE (Example 1 (c)) was also used to assess the reaction, cation exchange purification fractions, (Figure 3), and final purified products. SDS-PAGE was carried out on 1 mm thick 10-NuPAGE gels (Invitrogen, Carlsbad, CA) under reducing and non-reducing conditions and stained using a Novex Colloidal Coomassie^{™} G-250 staining kit (Invitrogen, Carlsbad, CA).

### Biological Assays

The utility of the PYY agonists of the present invention as pharmaceutically active agents in the reduction of weight gain and treatment of obesity in mammals (particularly, humans), may be demonstrated by the activity of the agonists in conventional assays and in the *in vitro* and *in vivo* assays described below. Such assays also provide a means whereby the activities of the present PYY agonists can be compared with the activities of known compounds.

### Food Intake Studies

**Fasting-induced refeeding assay:** C57BU6J male mice (The Jackson Laboratory, Bar Harbor, ME) were housed 2 per cage. They were maintained on a 12:12 light:dark cycle (lights on at 5:00 AM, lights off at 5:00 PM), fed pelleted RMH3000 Purina rodent chow (Research Diets, Inc., New Brunswick, NJ), and allowed water ad lib. The mice arrived at 7-8 weeks of age and were acclimated a minimum of 10 days prior to study. On the day of study, mice were 9-12 weeks old. The day prior to starting the study, the mice were placed into cages with fresh bedding and no food, but allowed free access to water. They were fasted overnight (20-24 hrs). The day of the study, mice were dosed IP injection (dose volume = 5 mL/kg), returned to their cage, and pre-weighed food was immediately placed in the cage. The dosing vehicle used was 20 mM Na acetate, pH 4.5, 50 mM NaCl and dose was calculated for the active PYY entity without pegylation. Vehicle control, native PYY, 30K mPEG maleimide (E10C)hPYY₃₋₃₆, and 43K mPEG maleimide(E10C)hPYY₃₋₃₆ at three doses (0.1 mg/kg, 0.3 mg/kg, and 1.0 mg/kg) were tested. Food was reweighed at 2, 4, 6, and 24 hours post-dosing. Bedding was checked for spillage, which was weighed and included in the calculations. Cumulative food intake was calculated by subtracting the food weight at each time point from the starting food weight. Percent (%) inhibition was calculated by (FIₜᵣₑₐₜ - FIᵥₑₕ)/FIᵥₑₕ * 100.

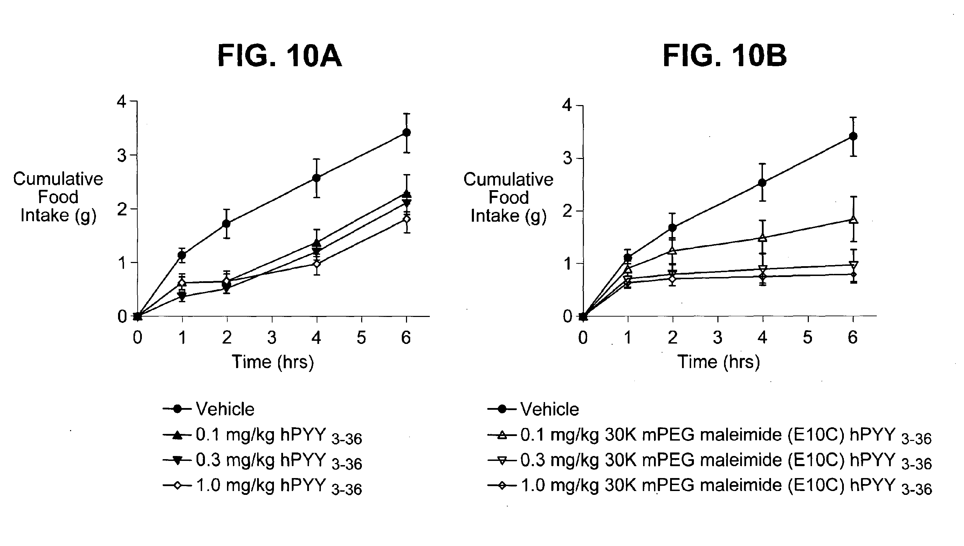

Figure 10 shows the 6-hour cumulative intake following the IP injection the three doses of native PYY₃₋₃₆ (Figure 10A) and the 30K mPEG maleimide(E10C)hPYY₃₋₃₆ (Figure 10B). Both native PYY₃₋₃₆ and the 30K mPEG maleimide(E10C)hPYY₃₋₃₆ demonstrated a dose-dependent decrease in cumulative food intake over the course of 6 hours.

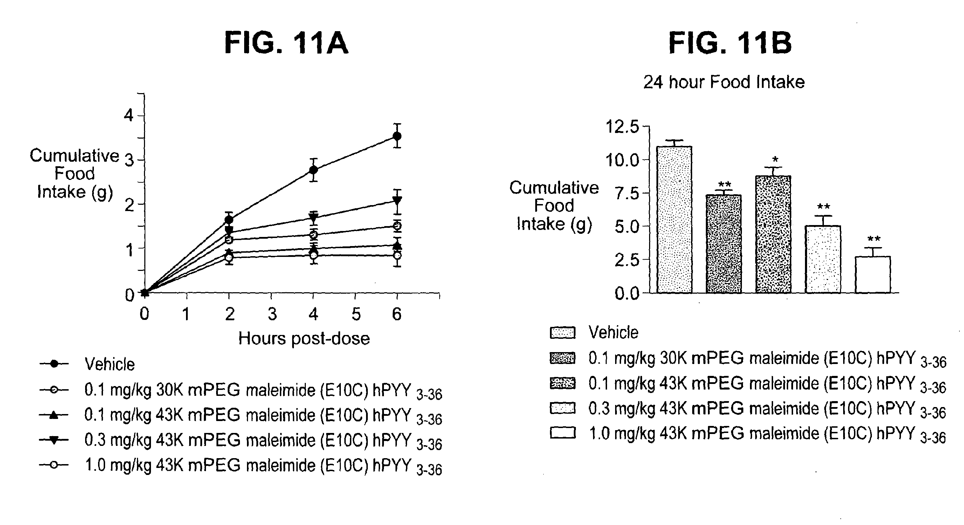

The 43K mPEG maleimide(E10C)hPYY₃₋₃₆ also produced a dose dependent decrease in 6 hour (Figure 11A) and 24 hour (Figure 11B) cumulative food intake. However, the 43K mPEG maleimide(E10C)hPYY₃₋₃₆ effect to reduce cumulative food intake was not as great as that demonstrated by the 30K mPEG maleimide(E10C)hPYY₃₋₃₆ at the same dose (0.1 mg/kg) after both 6 hours and 24 hours.

The effects of 30K mPEG maleimide(E10C)hPYY₃₋₃₆ on fasting-induced refeeding following injection of 0.1 mg/kg (SC) were also compared to the effects of 30K maleimide(D11C)hPYY₃₋₃₆. While the 30K mPEG maleimide(D11C)hPYY₃₋₃₆ polypeptide did cause reduced cumulative food intake (FI) over the 24 hour time course, as shown in the table below, the effect was not as great as that observed for the 30K maleimide(E10C)hPYY₃₋₃₆.

| Treatment | | 2hr FI | % change | 4hr FI | % change | 6hr FI | % change | 24hr FI | |
|---|---|---|---|---|---|---|---|---|---|
| Vehicle | Mean | 2.03 | 0.00 | 2.93 | 0.00 | 4.06 | 0.00 | 10.79 | 0.00 |
| | SEM | 0.16 | 8.06 | 0.22 | 7.46 | 0.46 | 11.30 | 4.67 | 6.23 |
| | | | | | | | | | |
| E10C | Mean | 1.89 | -6.99 | 2.16 | -26.21 | 2.45 | -39.62 | 8.04 | -25.48 |
| | SEM | 0.27 | 13.45 | 0.27 | 9.09 | 0.26 | 6.33 | 0.88 | 8.14 |
| | | | | | | | | | |
| D11C | Mean | 2.14 | 5.22 | 2.56 | -12.56 | 3.09 | -24.02 | 9.08 | -15.88 |
| | SEM | 0.15 | 7.30 | 0.23 | 7.72 | 0.27 | 6.68 | 0.42 | 3.88 |

The effects of linear 20K mPEG maleimide(E10C)hPYY₃₋₃₆ were compared to 30K mPEG maleimide(E10C)hPYY₃₋₃₆ (both of Example 1). In one study in which a dose of 0.1 mg/kg (IP) was injected in male mice, the results are as follows in the table below.

**% change in cumulative food intake versus vehicle-treated**

| Treatment | 2hr FI | 4hr FI | 6hr FI | 24hr FI | 48hr FI |
|---|---|---|---|---|---|
| 30K E10C | -56 | -65 | -78 | -47 | -20 |
| 20K E10C | -65 | -73 | -79 | -36 | -17 |

Similarly, in a second study comparing the feeding effects of linear 20K mPEG maleimide(E10C)hPYY₃₋₃₆ 30K mPEG maleimide(E10C)hPYY₃₋₃₆, following a 0.1 mg/kg dose (SC), the results are as follows in the table below.

**% change in cumulative food intake versus vehicle-treated**

| Treatment | 2hr FI | 4hr FI | 6hr FI | 24hr FI | 48hr FI | 72hr FI |
|---|---|---|---|---|---|---|
| 30K E10C | -13 | -24 | -29 | -20 | -12 | -2 |
| 20K E10C | -45 | -55 | -58 | -28 | -14 | -5 |

**Plasma PYY concentrations following SC injection were as follows.**

| Treatment | 2hr | 4hr | 6hr | 24hr | 30hr | 48hr |
|---|---|---|---|---|---|---|
| 30K E10C | 151±58 | 204±48 | 308±29 | 139±27 | 79±14 | 40±6 |
| 20K E10C | 110±21 | 186±37 | 204±14 | 62±16 | 45±12 | 13±3 |

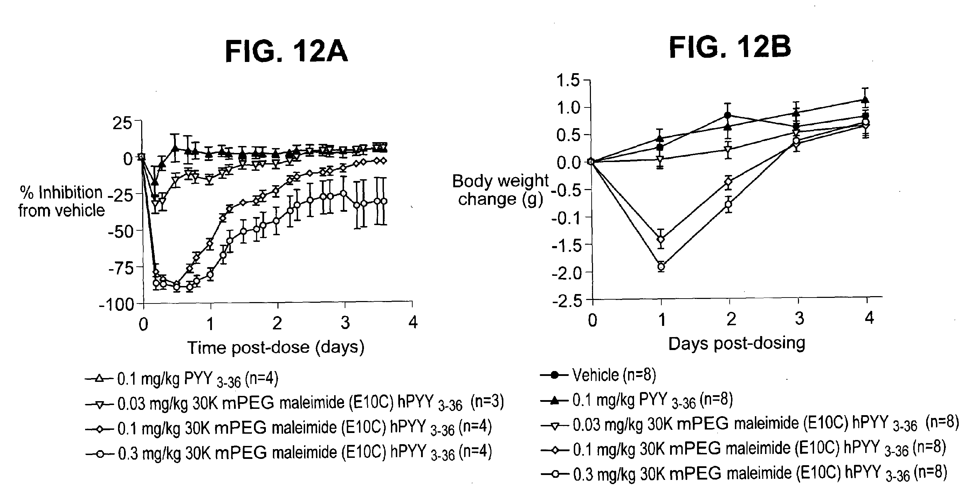

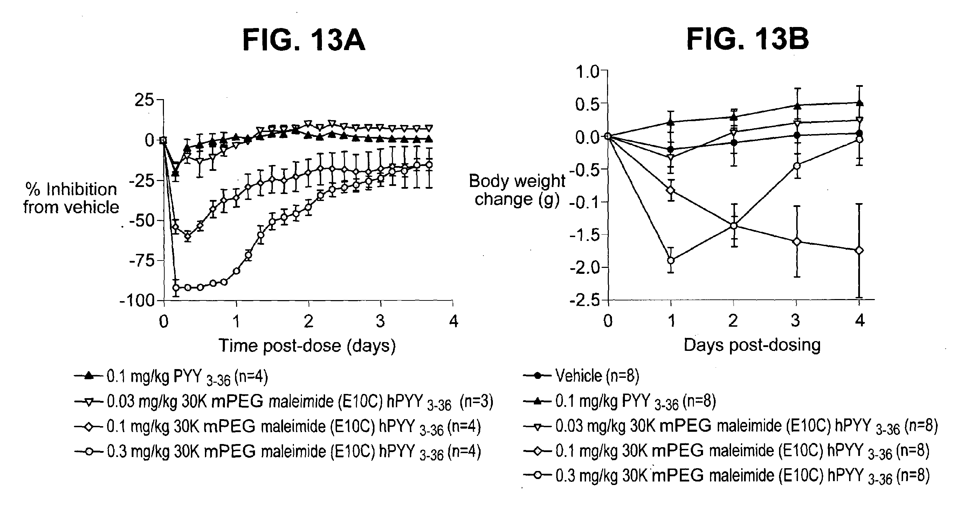

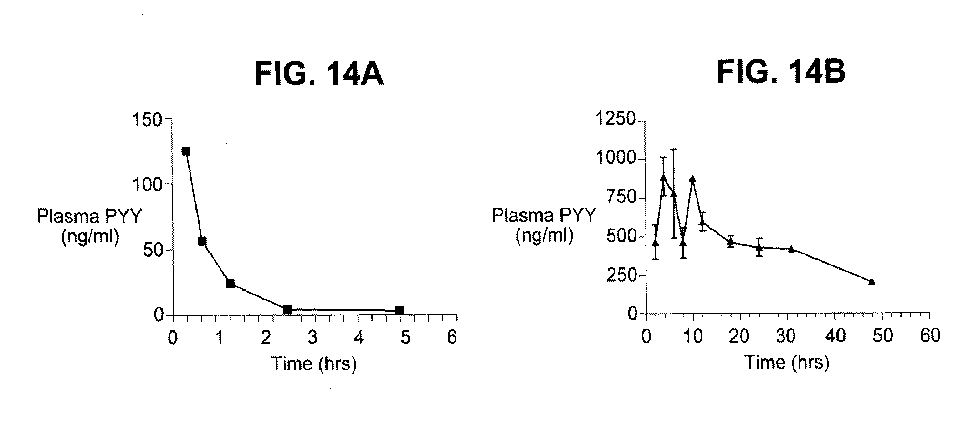

**Spontaneous food intake assay:** C57BL/6J male mice (The Jackson Laboratory) were individually housed and allowed 2 weeks acclimation before the study. They were maintained on a 12/12 light/dark cycle, fed powdered chow ad lib, and allowed free access to water. On the day before dosing, the mice were placed in food intake chambers, and allowed 1 day acclimation. The following day, the mice were dosed with IP or subcutaneous (SC) injection just prior to lights out (4:00 PM). Food intake was automatically monitored at 10 minute intervals throughout the entire timecourse and body weights were measured daily. Results are shown for IP injection of native PYY₃₋₃₆ and the 30K mPEG maleimide(E10C)hPYY₃₋₃₆ (Figure 12) and for SC injection of native PYY₃₋₃₆ and the 30K PEG maleimide(E10C)hPYY₃₋₃₆ (Figure 13). While both native PYY₃₋₃₆ and the 30K mPEG maleimide(E10C)hPYY₃₋₃₆ produced an immediate reduction in cumulative food intake as compared to vehicle-treated mice, the reduced food intake effect caused by the 30K mPEG maleimide(E10C)hPYY₃₋₃₆ was of much longer duration that the effect caused by the native PYY₃₋₃₆. Coupled with a more lasting food intake effect, the 30K mPEG maleimide(E10C)hPYY₃₋₃₆ also demonstrated a prolonged plasma exposure following the single injection (0.1 mg/kg, IP) (Figure 14). While native PYY₃₋₃₆ had a clearance rate of 16 ml/min/kg and a Cₘₐₓ of 38 nM, the 30K mPEG maleimide(E10C)hPYY₃₋₃₆ had a clearance rate of 0.2 ml/min/kg and Cₘₐₓ of 267 nM. Plasma PYY values were measured in mice for using an hPYY radioimmunoassay kit (Linco Research, Inc., St. Louis, MO).

**Mini-pump assay with ob/ob mice**: Male ob/ob mice (The Jackson Laboratory), 8-9 weeks of age (n=26), were maintained on normal chow and implanted with 14-day osmotic mini-pumps (Alza Corp., Mountain View, CA) which administered either vehicle (saline), PYY₃₋₃₆ (0.1 mg/kg/day), or 30K PEG maleimide(E10C)hPYY₃₋₃₆ (0.03 mg/kg/day). Food weights and body weights were measured daily. Body fat composition was determined on day 0 and day 13. Blood samples were taken at the end of the study. There were no significant differences in food intake, body weight, or body fat composition for these groups. Plasma PYY was determined at termination of the study by radioimmunoassay as previously described. In the native PYY₃₋₃₆ treated group, plasma PYY levels were measured at 15±2 ng/ml; in the 30K mPEG maleimide(E10C)hPYY₃₋₃₆ treated group, plasma PYY levels were 132±22 ng/ml.

### In vitro binding studies

### SPA for ligand binding:

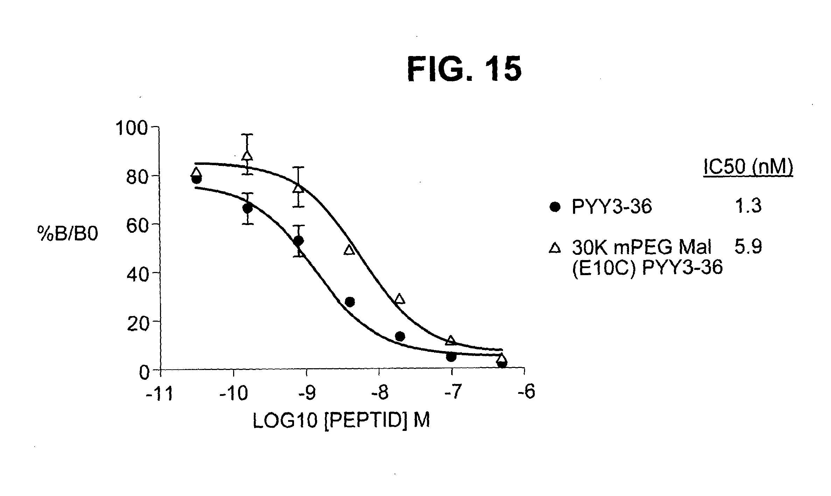

The SPA for ligand binding measures the competitive displacement of radiolabeled PYY from Y2 receptors and utilizes microspheres containing scintillant (SPA beads) coated with lectin wheat germ agglutinin (WGA) obtained from Amersham Biosciences (Cat. No. RPNQ 0085). Suspensions of KAN-TS human neuroblastoma cells that express Y2 receptors on their surface (Fuhlendorf et al., Proc. Natl. Acad. Sci. USA, 87: 182-186, 1990) were prepared using a cell harvesting buffer composed of 50 mM Hepes buffer (pH 7.4), 145 mM NaCl, 2.5 mM CaCl₂, 1 mM MgCl₂, 10 mM glucose, 0.1% BSA, 5% DMSO and Roche protease inhibitors. SPA assays were performed in 96-well format in triplicate using 50, 000 cells/well, ¹²⁵I-PYY (40,000 cpm/ well) and SPA beads (0.5mg/well) in assay buffer composed of 50mM Hepes buffer, pH 7.4, 1mM MgCl₂, 2.5mM CaCl₂, 0.1% (w/v) BSA, 0.025% (w/v) bacitracin and 0.025% sodium azide. Test ligands at various concentrations (0.032 to 500nM) were added to the assay mix which was then incubated for 16-24h at room temperature, while shaking. The plates were allowed to stand for one hour and then counted using a MicroBeta^{®} Trilux detector (Perkin Elmer, Boston, MA). Results for hPYY₃₋₃₆ and the 30K mPEG maieimide(E10C)hPYY₃₋₃₆ of Example 1 are shown in Figure 15.

### GTPγ [³⁵S] binding assays at NPY Y2R receptors

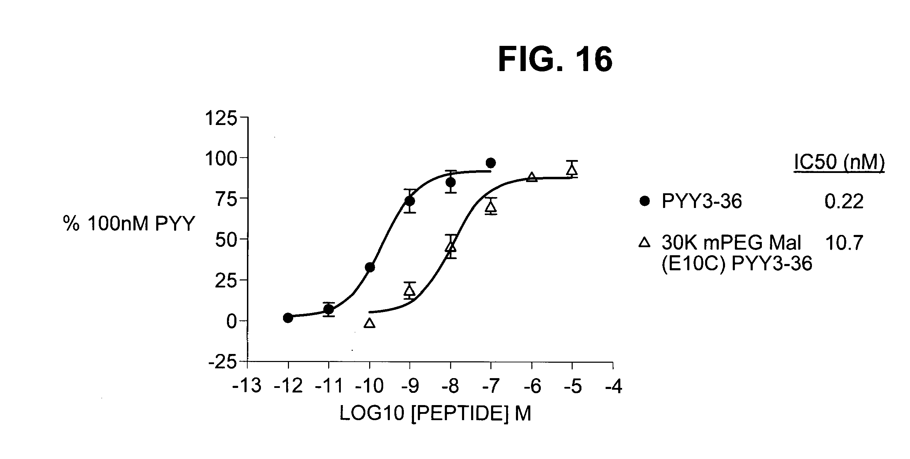

The functional assay is a GTPγ [³⁵S] binding assay run in NEN Flashplates (96-well format). Membranes were prepared from KAN-TS cells as described in Bass et al., Mol. Pharm. 50: 709-715, 1990. GTPγ [³⁵S] binding assays were performed in a 96 well FlashPlate^{™} format in duplicate using 100 pM GTPγ [³⁵S] and 10 µg membrane per well in assay buffer composed of 50 mM Tris Hcl, pH 7.4, 3 mM MgCl₂, pH 7.4, 10 mM MgCl₂, 20 mM EGTA, 100 mM NaCl, 5 µM GDP, 0.1 % bovine serum albumin and the following protease inhibitors: 100 µg/mL bacitracin, 100 µg/mL benzamidine, 5 µg/mL aprotinin, 5 µg/mL leupeptin. The assay mix was then incubated with increasing concentrations of test compound (6-point concentration curve; log dilutions in the range of 10⁻¹² M to 10⁻⁵ M) for 60 min. at 30° C. The FlashPlates^{™} were then centrifuged at 2000Xg for 10 minutes. Stimulation of GTPγ [³⁵S] binding was then quantified using a Microbeta^{™} detector. EC₅₀ and intrinsic activity calculations calculated using Prism by Graphpad. Results for hPYY₃₋₃₆ and the 30K mPEG maleimide(E10C)hPYY₃₋₃₆ of Example 1 are shown in Figure 16. EC₅₀ values for the 30K mPEG maleimide(E10C)hPYY₃₋₃₆ and the 20K mPEG maleimide(E10C)hPYY₃₋₃₆ of Example 1 were comparable (e.g., 4.3 nM and 4.6 nM when measured in the same assay).

## Claims

1. A PYY agonist variant of a mammalian PYY₃₋₃₆ in which residue 10 (glutamic acid) or residue 11 (aspartic acid) has been replaced with an amino acid "X" which is selected from the group consisting of lysine, serine, threonine, tyrosine, and unnatural amino acids, having a functionality that is conjugatable with a hydrophilic polymer.

2. The variant of claim 1, wherein said hydrophilic polymer is polyethylene glycol (PEG).

3. The variant of claim 1 or claim 2, wherein said functionality is a keto, thiol, hydroxyl, carboxyl, or free amino functionality.

4. The variant of any one of claims 1 to 3, which is designated (E10X)PYY₃₋₃₆ or (D11X)PYY₃₋₃₆, respectively.

5. The variant of any one of claims 1 to 4, wherein said variant of a mammalian PYY₃₋₃₆ is human PYY₃₋₃₆, canine PYY₃₋₃₆, feline PYY₃₋₃₆, or equine PYY₃₋₃₆.

6. The variant of claim 5, wherein said variant of a mammalian PYY₃₋₃₆ is human PYY₃₋₃₆.

7. The variant of claim 6, wherein said human PYY₃₋₃₆ has the amino acid sequence:
YPIKPEAPGEDASPEELNRYYASLRHYLNLVTRQRY-NH₂ [SEQ ID NO:1].

8. The variant of any one of claims 2 to 7, wherein said variant is pegylated.

9. The variant of claim 8, wherein said variant is monopegylated.

10. The variant of any one of claims 2 to 9, wherein said PEG is linear, branched, or pendant.

11. A pharmaceutical composition comprising the variant of any one of claims 1 to 10 and a pharmaceutically acceptable carrier.

12. The pharmaceutical composition of claim 11, further comprising a second agent that is an anti-obesity agent.

13. The use of the variant of any one of claims 1 to 10 or the pharmaceutical composition of any one of claims 11 or 12 in the manufacture of a medicament for treating obesity in a mammal, treating a condition of being overweight in a mammal, reducing weight or promoting weight loss (including preventing or inhibiting weight gain) in a mammal, reducing food intake in a mammal, inducing satiety in a mammal, or reducing caloric intake in a mammal.

14. The variant of any one of claims 1 to 10 or the pharmaceutical composition of any one of claims 11 or 12 for use in treating obesity in a mammal, treating a condition of being overweight in a mammal, reducing weight or promoting weight loss (including preventing or inhibiting weight gain) in a mammal, reducing food intake in a mammal, inducing satiety in a mammal, or reducing caloric intake in a mammal.

15. A polynucleotide encoding the (polypeptide) variant of claim 1.

16. A monoclonal antibody that specifically binds to a polypeptide comprising the amino acid sequence as shown in SEQ ID NO:1


SEQUENCE LISTING

<110>  PFIZER PRODUCTS INC
       Summers, Neena L.
       Finn, Rory F.
       Siegel, Ned R.
         Nardone, Nancy A.

<120>  PYY AGONISTS AND USES THEREOF

<130>  PC32768A-DIV-EP

<150>  US 60/650,366
<151>  2005-02-04

<150>  US 60/733,656
<151>  2005-11-04

<160>  8

<170>  PatentIn version 3.3

<210>  1
<211>  36
<212>  PRT
<213>  Homo sapiens

<400>  1

Tyr Pro Ile Lys Pro Glu Ala Pro Gly Glu Asp Ala Ser Pro Glu Glu
1               5                   10                  15

Leu Asn Arg Tyr Tyr Ala Ser Leu Arg His Tyr Leu Asn Leu Val Thr
            20                  25                  30

Arg Gln Arg Tyr
        35

<210>  2
<211>  34
<212>  PRT
<213>  Homo sapiens

<400>  2

Ile Lys Pro Glu Ala Pro Gly Glu Asp Ala Ser Pro Glu Glu Leu Asn
1               5                   10                  15

Arg Tyr Tyr Ala Ser Leu Arg His Tyr Leu Asn Leu Val Thr Arg Gln
            20                  25                  30

Arg Tyr

<210>  3
<211>  34
<212>  PRT
<213>  Homo sapiens

<400>  3

Ile Lys Pro Glu Ala Pro Gly Cys Asp Ala Ser Pro Glu Glu Leu Asn
1               5                   10                  15

Arg Tyr Tyr Ala Ser Leu Arg His Tyr Leu Asn Leu Val Thr Arg Gln
            20                  25                  30

Arg Tyr

<210>  4
<211>  34
<212>  PRT
<213>  Homo sapiens

<400>  4

Ile Lys Pro Glu Ala Pro Gly Glu Cys Ala Ser Pro Glu Glu Leu Asn
1               5                   10                  15

Arg Tyr Tyr Ala Ser Leu Arg His Tyr Leu Asn Leu Val Thr Arg Gln
            20                  25                  30

Arg Tyr

<210>  5
<211>  102
<212>  DNA
<213>  Homo sapiens

<400>  5
atcaaacccg aggctcccgg ctgtgacgcc tcgccggagg agctgaaccg ctactacgcc      60

tccctgcgcc actacctcaa cctggtcacc cggcagcggt at                        102

<210>  6
<211>  102
<212>  DNA
<213>  Homo sapiens

<400>  6
atcaaacccg aggctcccgg ctgcgacgcc tcgccggagg agctgaaccg ctactacgcc      60

tccctgcgcc actacctcaa cctggtcacc cggcagcggt at                        102

<210>  7
<211>  102
<212>  DNA
<213>  Homo sapiens

<400>  7
atcaaacccg aggctcccgg cgaatgtgcc tcgccggagg agctgaaccg ctactacgcc      60

tccctgcgcc actacctcaa cctggtcacc cggcagcggt at                        102

<210>  8
<211>  102
<212>  DNA
<213>  Homo sapiens

<400>  8
atcaaacccg aggctcccgg cgaatgcgcc tcgccggagg agctgaaccg ctactacgcc      60

tccctgcgcc actacctcaa cctggtcacc cggcagcggt at                        102